(19) Europäisches Patentamt
European Patent Office
Office européen des brevets

(11) **EP 2 937 033 A1**

(12) **EUROPEAN PATENT APPLICATION**
published in accordance with Art. 153(4) EPC

(43) Date of publication:
**28.10.2015 Bulletin 2015/44**

(51) Int Cl.:
*A61B 1/00* *(2006.01)*     *G02B 23/24* *(2006.01)*
*G06T 1/00* *(2006.01)*     *H04N 7/18* *(2006.01)*

(21) Application number: **13864535.3**

(22) Date of filing: **25.09.2013**

(86) International application number:
**PCT/JP2013/075870**

(87) International publication number:
**WO 2014/097702 (26.06.2014 Gazette 2014/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.12.2012 JP 2012278216**
**26.03.2013 JP 2013065117**

(71) Applicant: **OLYMPUS CORPORATION**
**Shibuya-ku**
**Tokyo 151-0072 (JP)**

(72) Inventor: **SASAKI, Hiroshi**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **IMAGE PROCESSING APPARATUS, ELECTRONIC DEVICE, ENDOSCOPE APPARATUS, PROGRAM, AND IMAGE PROCESSING METHOD**

(57) An image processing device includes an image acquisition section (390) that acquires a captured image that includes an image of an object, the captured image being an image captured by an imaging section (200), a distance information acquisition section (340) that acquires distance information based on the distance from the imaging section (200) to the object when the imaging section (200) captured the captured image, a known characteristic information acquisition section (350) that acquires known characteristic information, the known characteristic information being information that represents known characteristics relating to the structure of the object, and a concavity-convexity determination section (310) that performs a concavity-convexity determination process that specifies an concavity-convexity part of the object that agrees with the characteristics specified by the known characteristic information, from the object captured within the captured image, based on the distance information and the known characteristic information.

FIG. 18A

# FIG. 18B

CALCULATE NORMAL VECTOR WITH RESPECT TO RESULTS OF CLOSING PROCESS

NORMAL VECTOR

**Description**

TECHNICAL FIELD

The present invention relates to an image processing device, an electronic device, an endoscope apparatus, a program, an image processing method, and the like.

BACKGROUND ART

When observing tissue using an endoscope apparatus, and making a diagnosis, a method has been widely used that determines whether or not an early lesion has occurred by observing the surface of tissue as to the presence or absence of minute concavities-convexities (concavity-convexity parts). When using an industrial endoscope apparatus instead of a medical endoscope apparatus, it is useful to observe the object (i.e., the surface of the object in a narrow sense) as to the presence or absence of an concavity-convexity structure in order to detect whether or not a crack has occurred in the inner side of a pipe that is difficult to directly observe with the naked eye, for example. It is normally useful to detect the presence or absence of a concavity-convexity structure from the processing target image (object) when using an image processing device other than an endoscope apparatus.

A process that enhances a specific spatial frequency has been widely used as a process for enhancing a structure (e.g., a concavity-convexity structure such as a groove) within the captured image. However, this method is not suitable for detecting the presence or absence of minute concavities-convexities (see above). A method has also been known that effects some change in the object, and captures the object, instead of detecting the presence or absence of concavities-convexities by image processing. For example, when using a medical endoscope apparatus, the contrast of the mucous membrane in the surface area may be increased by spraying a dye (e.g., indigocarmine) to stain the tissue. However, it takes time and cost to spray a dye, and the original color of the object, or the visibility of a structure other than concavities-convexities, may be impaired due to the sprayed dye. Moreover, the method that sprays a dye to tissue may be highly invasive for the patient.

Patent Document 1 discloses a method that enhances a concavity-convexity structure by comparing the luminance level of an attention pixel in a locally extracted area with the luminance level of its peripheral pixel, and coloring the attention area when the attention area is darker than the peripheral area.

Specific examples of the concavity-convexity structure of tissue include a ductal structure (pit pattern) present on the surface of tissue. For example, the pit pattern has been used to diagnose an early lesion in the large intestine. This diagnostic method is referred to as "pit pattern diagnosis". The pit patterns are classified into six types (type I to type V) corresponding to the type of lesion, and the pit pattern diagnosis determines the type into which the observed pit pattern is classified.

Patent Document 2 discloses a device that acquires a three-dimensional optical tomographic image using an endoscope and an optical probe, and discloses a method that samples XY plane images perpendicular to the depth direction of tissue at a plurality of depth positions based on the three-dimensional optical tomographic image, and enhances the pit pattern based on the average image.

RELATED-ART DOCUMENT

PATENT DOCUMENT

Patent Document 1: JP-A-2003-88498
Patent Document 2: JP-A-2010-68865

SUMMARY OF THE INVENTION

TECHNICAL PROBLEM

The process disclosed in Patent Document 1 is designed based on the assumption that the object (i.e., the surface of tissue) is captured darkly when the distance from the imaging section to the object is long, since the intensity of reflected light from the surface of the tissue decreases. Therefore, a concavity-convexity structure may be erroneously detected in an area in which a change in luminance occurs irrespective of minute concavities-convexities that may be present on the surface of tissue (e.g., an area around a bright spot, a shadow area that is covered by the front structure, a blood vessel, or a mucous membrane around a blood vessel).

The pit pattern diagnosis is performed in a state in which an area that may be a lesion has been found by screening observation, and is closely observed by bringing the end of the endoscope closer to the area. Since the magnification of the captured image of the surface of tissue (observation target) increases during close observation and zoom observation, the effect of the relative motion of the tissue and the imaging section increases. In particular, since it is necessary to cause the optical probe to make a scan motion when acquiring a three-dimensional optical tomographic image using the method disclosed in Patent Document 2, deformation of the pit shape within the XY image may significantly occur due to the relative motion of the tissue and the imaging section. Therefore, it may be difficult to stably calculate the similarity with the model pattern used to detect the pit pattern, and acquire an accurate image.

Several aspects of the invention may provide an image processing device, an electronic device, an endoscope apparatus, a program, and an image processing method that accurately detect an concavity-convexity part of the object based on known characteristic information and distance information about the object.

SOLUTION TO PROBLEM

According to one aspect of the invention, there is provided an image processing device comprising:

an image acquisition section that acquires a captured image that includes an image of an object, the captured image being an image captured by an imaging section;

a distance information acquisition section that acquires distance information based on a distance from the imaging section to the object when the imaging section captured the captured image;

a known characteristic information acquisition section that acquires known characteristic information, the known characteristic information being information that represents known characteristics relating to a structure of the object; and

a concavity-convexity determination section that performs a concavity-convexity determination process that specifies an concavity-convexity part of the object that agrees with characteristics specified by the known characteristic information, from the object captured within the captured image, based on the distance information and the known characteristic information.

According to one aspect of the invention, the distance information about the distance to the object, and the known characteristic information are acquired, and a concavity-convexity part of the object that agrees with the characteristics specified by the known characteristic information is specified. This makes it possible to accurately detect a concavity-convexity part using the method that utilizes image processing, for example.

According to another aspect of the invention, there is provided an electronic device comprising the above image processing device.

According to another aspect of the invention, there is provided an endoscope apparatus comprising the above image processing device.

According to these aspects of the invention, since a concavity-convexity part of tissue can be specified within the captured image, it is possible to easily find a lesion, for example.

According to another aspect of the invention, there is provided a program that causes a computer to function as each section described above.

According to another aspect of the invention, there is provided an image processing method comprising:

acquiring a captured image that includes an image of an object, the captured image being an image captured by an imaging section;

acquiring distance information based on a distance from the imaging section to the object when the imaging section captured the captured image;

acquiring known characteristic information, the known characteristic information being information that represents known characteristics relating to a structure of the object; and

performing a concavity-convexity determination process that specifies an concavity-convexity part of the object that agrees with characteristics specified by the known characteristic information, from the object captured within the captured image, based on the distance information and the known characteristic information.

BRIEF DESCRIPTION OF DRAWINGS

FIG. 1 illustrates a system configuration example of an image processing device.
FIG. 2 illustrates a configuration example of an endoscope apparatus that includes an image processing device according to the first embodiment.

FIG. 3 illustrates a configuration example of an image processing section according to the first embodiment.

FIGS. 4A to 4F are views illustrating an extraction process according to the first embodiment.

FIG. 5 illustrates a configuration example of a distance information acquisition section and a concavity-convexity information extraction section according to the first embodiment.

FIG. 6 illustrates a configuration example of an endoscope apparatus that includes an image processing device according to the second embodiment.

FIG. 7 illustrates a configuration example of an image processing section according to the second embodiment.

FIGS. 8A to 8D are views illustrating an extraction process according to the second embodiment.

FIG. 9 illustrates a configuration example of a concavity-convexity information extraction section according to the second embodiment.

FIG. 10 illustrates a configuration example of an endoscope apparatus that includes an image processing device according to the third embodiment.

FIG. 11 illustrates a configuration example of an image processing section according to the third embodiment.

FIG. 12 illustrates a configuration example of a concavity-convexity information extraction section according to the third embodiment.

FIG. 13 illustrates a configuration example of a distance information acquisition section according to the second embodiment.

FIG. 14 illustrates a configuration example of an image recording-replay device that includes an image processing device according to the fourth embodiment, and a capsule endoscope.

FIG. 15 illustrates a configuration example of an image processing section according to the fourth embodiment.

FIG. 16 illustrates a configuration example of an image processing section according to the fifth embodiment.

FIG. 17A illustrates an example of the cross section of a ductal structure, and FIG. 17B illustrates an example of a ductal structure within a captured image.

FIGS. 18A and 18B are views illustrating a process that calculates surface shape information.

FIGS. 19A and 19B illustrate an example of a reference pattern and a corrected pattern.

FIG. 20 illustrates a configuration example of a surface shape calculation section.

FIG. 21 illustrates a configuration example of a classification processing section according to the fifth embodiment.

FIG. 22 illustrates an example of a classification map that is the results of a classification process.

FIG. 23 illustrates a configuration example of an endoscope apparatus that includes an image processing device according to the sixth embodiment.

FIG. 24 illustrates a configuration example of a classification processing section according to the sixth embodiment.

FIG. 25 illustrates an example when storing a plurality of reference patterns.

FIGS. 26A to 26D illustrate an example of a classification map that is the results of a classification process when using a plurality of reference patterns.

FIGS. 27A to 27F are views illustrating a similarity calculation process.

FIGS. 28A to 28F illustrate an example of a pit pattern.

DESCRIPTION OF EMBODIMENTS

Exemplary embodiments of the invention are described below. Note that the following exemplary embodiments do not in any way limit the scope of the invention laid out in the claims. Note also that all of the elements described in connection with the following exemplary embodiments should not necessarily be taken as essential elements of the invention.

1. Method

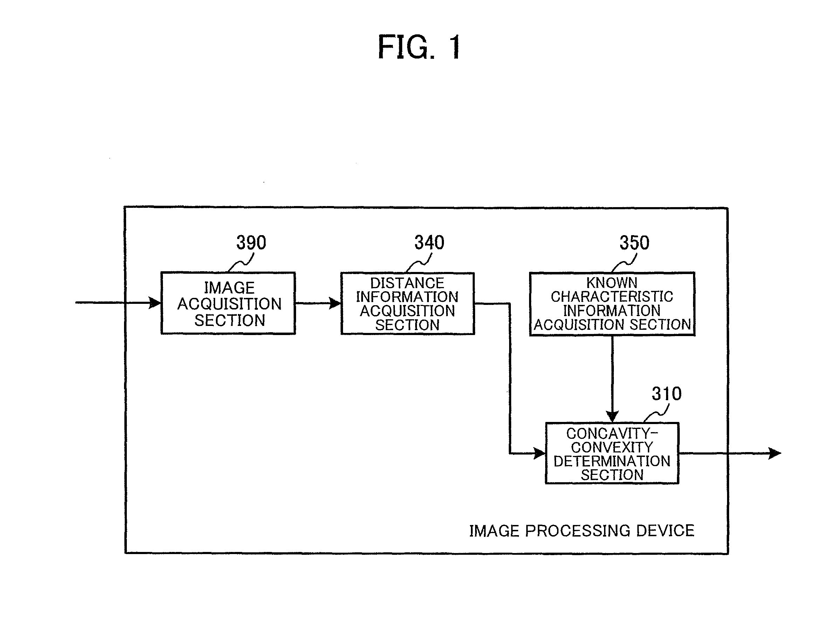

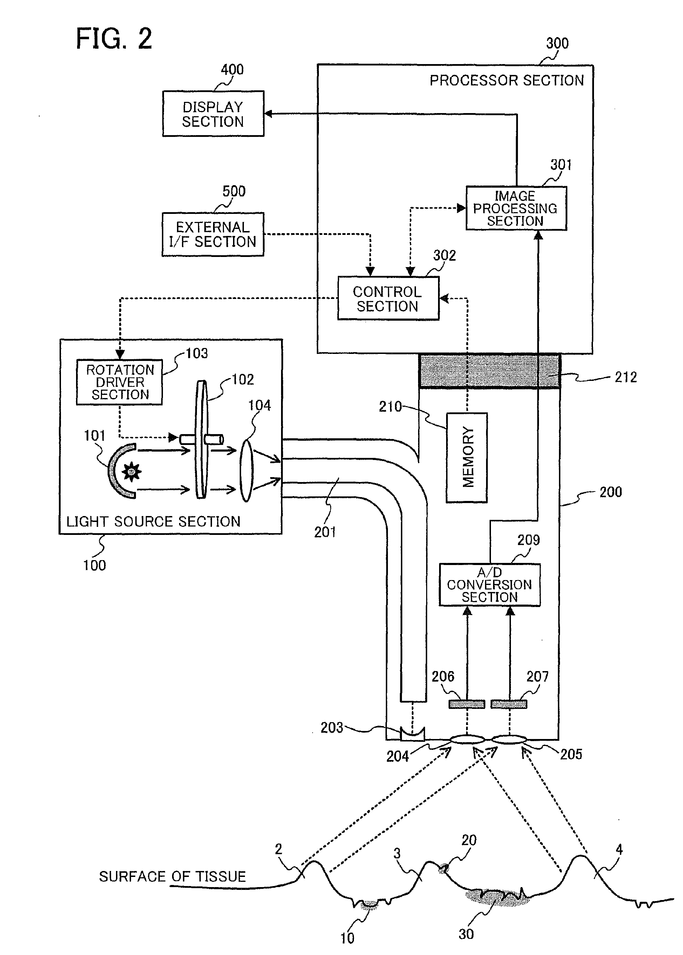

As illustrated in FIG. 1, an image processing device according to several embodiments of the invention includes an image acquisition section 390 that acquires a captured image that includes an image of an object, the captured image being an image captured by an imaging section (e.g., imaging section 200 illustrated in FIG. 2 (described later)), a distance information acquisition section 340 that acquires distance information based on the distance from the imaging section to the object when the imaging section captured the captured image, a known characteristic information acquisition section 350 that acquires known characteristic information, the known characteristic information being information that represents known characteristics relating to the structure of the object, and a concavity-convexity determination section 310 that performs a concavity-convexity determination process that specifies an concavity-convexity part of the object that agrees with characteristics specified by the known characteristic information, from the object captured within the captured image, based on the distance information and the known characteristic information.

The concavity-convexity part that is specified by the concavity-convexity determination section 310 may be a minute concavity-convexity structure (e.g., groove or polyp) that has given dimensions (e.g., width, depth, or height) specified by the known characteristic information, or may be a ductal structure (pit pattern) present on the surface of tissue.

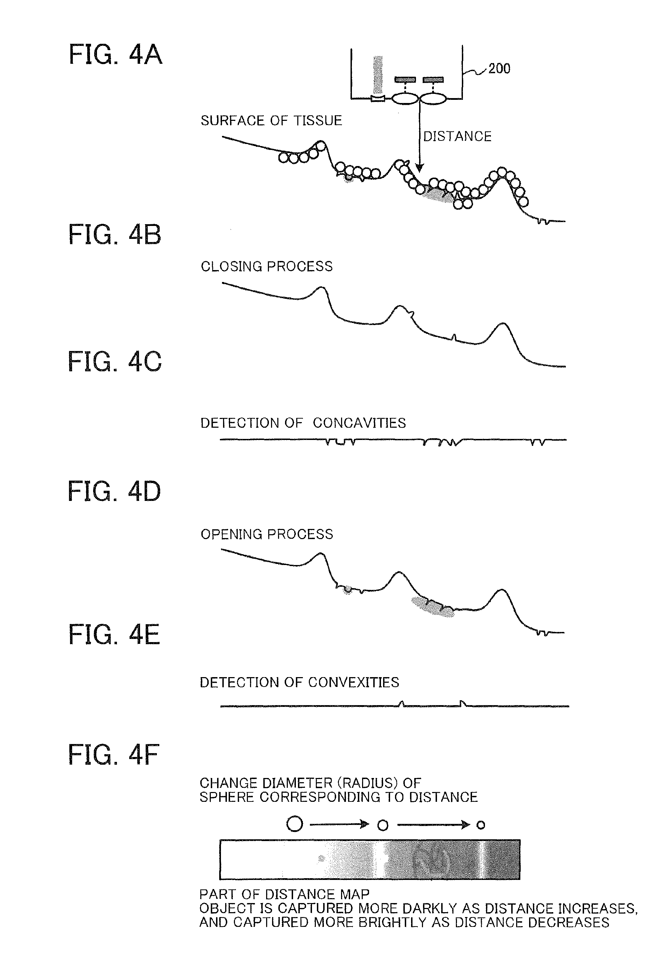

An example in which the concavity-convexity determination section 310 specifies a minute concavity-convexity structure is described below. Since the distance information that is acquired by the distance information acquisition section 340 is information that corresponds to the distance from the imaging section to the object, the distance information represents the structure of the object (i.e., tissue (particularly the surface of tissue) when using a medical endoscope apparatus) (see FIG. 4A). Specifically, the distance information includes information about a minute concavity-convexity structure present on the surface of the object.

However, the distance information also includes information about a structure other than the minute concavity-convexity structure that is present on the surface of the object. For example, a lumen structure (hollow tubular structure) (e.g., gullet or large intestine) is normally observed using an endoscope apparatus. In this case, since the wall surface of the structure (tissue) forms a curved surface having a given curvature, the distance represented by the distance information varies corresponding to the curved surface. In the example illustrated in FIG. 4A, the distance information includes information about various structures, but represents a structure as a whole in which the distance from the imaging section to the object increases in the rightward direction.

The surface of the object may also include a concavity-convexity structure that differs from the concavity-convexity structure that is specified using the method according to several embodiments of the invention. For example, a fold structure (see 2, 3, and 4 in FIG. 2) may be observed on the surface of the stomach, the large intestine, or the like. The distance information also includes information about such a fold structure. Note that several embodiments of the invention are intended to observe (using an endoscope apparatus) a minute concavity-convexity structure that differs in dimensions from such a structure that is normally observed on the surface of tissue.

Therefore, it is necessary to appropriately extract the information about the desired concavity-convexity structure from the distance information that includes a change in distance due to various structures in order to appropriately specify a concavity-convexity part that is useful when performing an enhancement process and the like. This also applies to the case of using an industrial endoscope apparatus. When using an industrial endoscope apparatus, the distance information may include a change in distance that corresponds to the curved surface of a circular pipe, information about a groove that is formed in advance in order to provide a pipe with a given function, information about a scratch or the like that may be missed due to low severity, and the like. In this case, it is desirable to extract a useful concavity-convexity structure as the extracted concavity-convexity information while excluding such information.

Several embodiments of the invention propose a method that acquires the known characteristic information that is information that represents the known characteristics relating to the structure of the object, and specifies the concavity-convexity part of the object that agrees with the characteristics specified by the known characteristic information within the captured image. The term "known characteristic information" used herein refers to information that makes it possible to classify the structures of the surface of the object into a useful structure and a structure that is not useful. Specifically, information about the curvature of the wall surface of the tissue, dimensional information about a fold, and the like may be stored as the known characteristic information, information that agrees with the known characteristic information may be excluded from the distance information, and a concavity-convexity part may be specified by utilizing the resulting information as the extracted concavity-convexity information. The dimensional information about a useful concavity-convexity structure may be used as the known characteristic information. In this case, information that agrees with the known characteristic information is extracted from the distance information as the extracted concavity-convexity information, and a concavity-convexity part is specified based on the extracted concavity-convexity information. Specifically, the known characteristic information includes the information that corresponds to the exclusion target, and the information that corresponds to the extraction target. The following description illustrates an example that uses both the information that corresponds to the exclusion target, and the information that corresponds to the extraction target. The term "characteristics specified by the known characteristic information" used herein refers to characteristics that correspond to the extraction target, and do not correspond to the exclusion target. For example, the characteristics specified by the known characteristic information refer to characteristics that have a boundary value that can clearly separate the extraction target and the exclusion target (or a value within the range determined by the boundary value).

It is considered that a typical fold size, the dimensions of a useful concavity-convexity structure, and the like differ corresponding to the observation target part (e.g., stomach (upper digestive system) or large intestine (lower digestive system)). Therefore, it is desirable to provide the known characteristic information so that the known characteristic information can be selected or changed corresponding to the observation target, for example.

Even if the known characteristic information is acquired as information that represents the actual size (e.g., $\mu$m) of the object, it is necessary to perform a process that converts the size of the object into the size within the image (distance information). For example, the size of a fold structure (having a given actual size) within the image increases when the fold structure is captured at a position close to the imaging section, and decreases when the fold structure is captured at a position away from the imaging section. Therefore, the process is adaptively changed corresponding to the value (distance) represented by the distance information. Specifically, an extraction process parameter that is used when extracting the extracted concavity-convexity information from the distance information is adaptively controlled corresponding to the value represented by the distance information.

An example in which the concavity-convexity determination section 310 specifies a pit pattern as the concavity-convexity part is described below. Note that several embodiments of the invention can be widely applied to a method that specifies a structure using a matching process that utilizes a two-dimensional pattern, and a pattern other than a pit pattern may also be used.

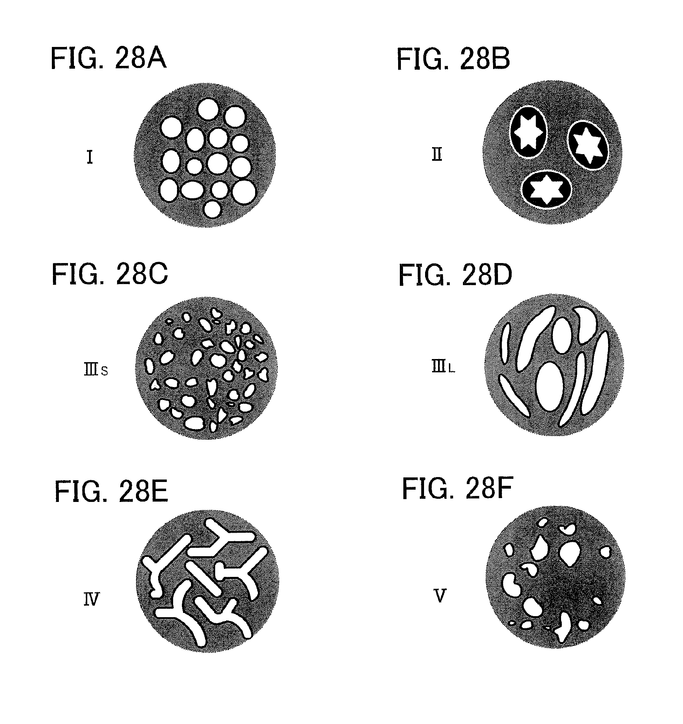

As illustrated in FIGS. 28A to 28F, the shape of a pit pattern on the surface of tissue changes corresponding to the state (normal state or abnormal state), the stage of lesion progression (abnormal state), and the like. For example, the pit pattern of a normal mucous membrane has an approximately circular shape (see FIG. 28A). The pit pattern has a complex shape (e.g., star-like shape (see FIG. 28B) or tubular shape (see FIGS. 28C and 28D) when a lesion has advanced, and may disappear (see FIG. 28F) when the lesion has further advanced. Therefore, it is possible to determine the state of the object by storing these typical patterns as a reference pattern, and determining the similarity between the surface of the object captured within the captured image and the reference pattern, for example. Patent Document 2 discloses a method that assists in such a pit pattern diagnosis, for example.

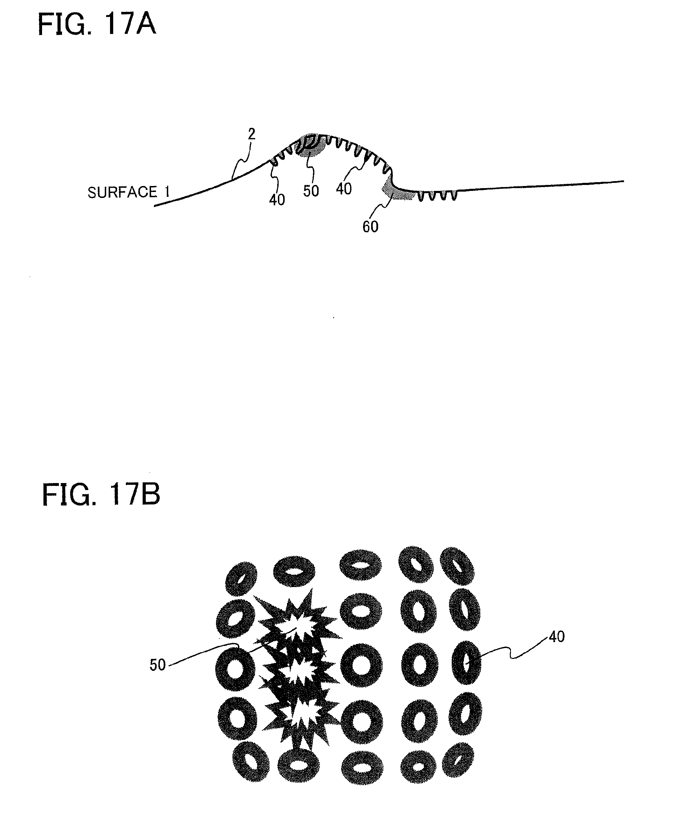

However, the pit pattern observed within the captured image does not necessarily coincide with the typical shape of the pit pattern. The wall surface of a lumen structure, and a structure such as a fold are observed on tissue. Therefore, the optical axis direction of the imaging section may not be orthogonal to the surface of tissue. In this case, a circular pit pattern present the surface of tissue may be observed as an elliptical pit pattern within the captured image, for example. In FIG. 17A, a fold 2 is present on the surface of tissue, and a circular pit pattern (normal duct 40) is observed on the surface of the fold 2, for example. In this case, the circular pit pattern is observed in a deformed state (see FIG. 17B) depending on the angle formed by the optical axis direction of the imaging section and the surface of the tissue.

As is clear from FIG. 17B, it is difficult to implement an accurate detection process in the area in which the pit pattern is deformed for the above reason, when a matching process is merely performed on the reference pattern and the captured image. Since the pit pattern diagnosis is performed during close observation or zoom observation, the effect of the relative motion of the imaging section and the tissue increases. When using the method disclosed in Patent Document 2, it is necessary to cause the optical probe to make a scan motion. Therefore, the pit pattern may be significantly deformed within the captured image.

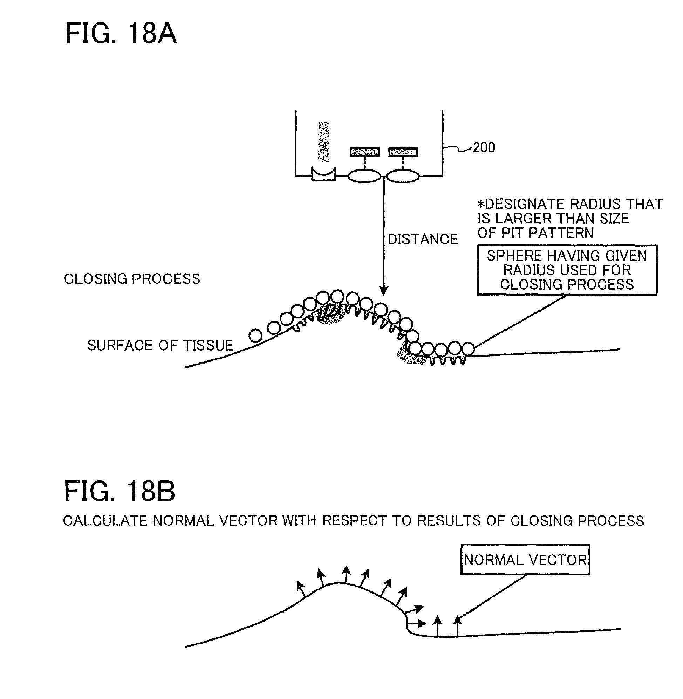

In order to deal with the above problem, several embodiments of the invention propose a method that acquires surface shape information that represents the structure present on the surface of the object based on the known characteristic information and the distance information, and specifies the concavity-convexity part using a classification process that utilizes a classification reference that is set using the surface shape information. The term "surface shape information" used herein refers to information that represents a global structure present on the surface of the object. For example, the surface shape information may be information that represents the curved surface illustrated in FIG. 18B (i.e., the distance information from which the minute concavity-convexity structure illustrated in FIG. 18A is excluded), or may be information that represents a set of the normal vectors to the curved surface.

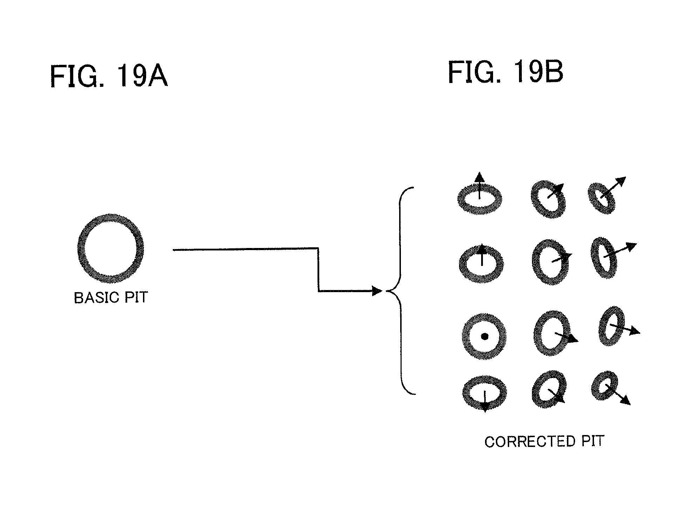

It is possible to estimate the deformation state of the reference pattern observed within the captured image by utilizing the surface shape information when the reference pattern is present on the surface of the object (processing target) (see FIGS. 19A and 19B). Specifically, whether or not a pit pattern corresponding to the reference pattern is observed on the surface of the object may be determined by performing the classification process using the pattern (hereinafter may be referred to as "corrected pattern") subjected to the deformation process using the surface shape information (see FIG. 19B) as the classification reference.

When the classification process is described taking a pit pattern as an example, the known characteristic information is information that represents a pit pattern (i.e., information that represents the pit shape, the pit size, and the like).

First to sixth embodiments of the invention are described below. Although the first to sixth embodiments are described below taking an endoscope apparatus (see FIG. 2) as an example, the first to sixth embodiments can be applied to an image processing device (see FIG. 1) that is not limited to an endoscope apparatus.

The first to fourth embodiments correspond to the method that specifies a minute concavity-convexity structure using the extracted concavity-convexity information. The first embodiment illustrates a method that acquires the distance information based on parallax information obtained from a plurality of captured images corresponding to a plurality of viewpoints, and extracts the extracted concavity-convexity information from the distance information using a morphological process. In the first embodiment, the extraction process parameter is the size of a structural element used for the morphological process. The second embodiment illustrates a method that acquires the distance information using the Time-of-Flight method, and extracts the extracted concavity-convexity information using a filtering process (particularly a low-pass filtering process). In the second embodiment, the extraction process parameter is a parameter that determines the frequency characteristics of a filter used for the filtering process.

The third embodiment illustrates a method that acquires the distance information by combining the method based on the parallax information obtained from a plurality of captured images corresponding to a plurality of viewpoints with the Time-of-Flight method, and extracts the extracted concavity-convexity information using a filtering process (particularly a high-pass filtering process). The fourth embodiment illustrates an example in which a capsule endoscope is used.

Note that the distance information acquisition process and the extracted concavity-convexity information extraction process may be combined in various ways. Specifically, the method based on the parallax information and the filtering process may be used in combination, or the Time-of-Flight method and the morphological process may be used in combination. The embodiments of the invention can be implemented by arbitrarily combining the above methods.

The fifth and sixth embodiments correspond to the method that specifies a concavity-convexity part (ductal structure in a narrow sense) by generating the classification reference using the surface shape information, and performing the classification process using the classification reference. Note that the concavity-convexity part is not limited to a ductal structure. The fifth embodiment illustrates a method that stores a pit pattern in a normal state as the reference pattern, and performs the classification process that determines whether or not each area of the object within the captured image is in a normal state.

The sixth embodiment illustrates a method that stores pit patterns that correspond to a plurality of states (e.g., a pit pattern in a normal state, and one or more pit patterns in an abnormal state) as the reference pattern, and performs the classification process that determines whether each area of the object within the captured image falls under the normal state or the abnormal state (or whether or not each area of the object within the captured image does not fall under the normal state and the abnormal state). The sixth embodiment also illustrates a method that acquires a second reference pattern from the captured image using the corrected pattern obtained by deforming the reference pattern, and uses a second corrected pattern obtained by deforming the second reference pattern using the surface shape information as the classification reference. It is expected that the detection accuracy can be further improved by calculating the classification reference from the captured object.

2. First embodiment

FIG. 2 illustrates a configuration example of an endoscope apparatus that includes the image processing device (corresponding to an image processing section 301) according to the first embodiment. The endoscope apparatus according to the first embodiment includes a light source section 100, an imaging section 200, a processor section 300, a display section 400, and an external I/F section 500.

The light source section 100 includes a white light source 101, a rotary color filter 102 that has a plurality of spectral transmittances, a rotation driver section 103 that drives the rotary color filter 102, and a condenser lens 104 that focuses light (that has passed through the rotary color filter 102, and has spectral characteristics) on the incident end face of a light guide fiber 201.

The rotary color filter 102 includes a red color filter, a green color filter, a blue color filter, and a rotary motor.

The rotation driver section 103 rotates the rotary color filter 102 at a given rotational speed in synchronization with the imaging period of image sensors 206 and 207 based on a control signal output from a control section 302 included in the processor section 300. For example, when the color filter is rotated at 20 revolutions per second, each color filter crosses the incident white light every 1/60th of a second, and the image sensors 206 and 207 capture reflected light (R, G, or B) from the observation target, and transfer the resulting image every 1/60th of a second. Specifically, the endoscope apparatus according to first embodiment frame-sequentially captures an R image, a G image, and a B image every 1/60th of a second, and the substantial frame rate is 20 fps.

The imaging section 200 is formed to be elongated and flexible (i.e., can be curved) so that the imaging section 200 can be inserted into a body cavity (e.g., stomach or large intestine), for example. The imaging section 200 includes the light guide fiber 201 that guides the light focused by the light source section 100, an illumination lens 203 that diffuses the light that has been guided by the light guide fiber 201, and applies the diffused light to the observation target, objective lenses 204 and 205 that focus the reflected light from the observation target, the image sensors 206 and 207 that detect the focused light, an A/D conversion section 209 that converts photoelectrically-converted analog signals output from the image sensors 206 and 207 into digital signals, a memory 210 that stores scope ID information and specific information (including a production variation) about the imaging section 200, and a connector 212 for removably connecting the imaging section 200 and the processor section 300. The image sensors 206 and 207 are monochrome single-chip image sensors, and may be implemented by a CCD sensor, a CMOS sensor, or the like.

The objective lenses 204 and 205 are disposed at a given interval so that a given parallax image (hereinafter referred to as "stereo image") can be captured. The objective lenses 204 and 205 respectively form a left image and a right image on the image sensors 206 and 207. The left image and the right image respectively output from the image sensors 206 and 207 are converted into digital signals by the A/D conversion section 209, and output to the image processing section 301. The memory 210 is connected to the control section 302, and the scope ID information and the specific information (including a production variation) are transmitted to the control section 302.

The processor section 300 includes the image processing section 301 and the control section 302.

The display section 400 is a display device (e.g., CRT or liquid crystal monitor) that can display a movie (moving image).

The external I/F section 500 is an interface that allows the user to input information to the endoscope apparatus, for example. The external I/F section 500 includes a power switch (power ON/OFF switch), a shutter button for starting imaging operation, a mode (e.g., imaging mode) switch button (e.g., a switch for selectively performing an enhancement process on a concavity-convexity part present on the surface of tissue), and the like. The external I/F section 500 outputs the input information to the control section 302.

In FIG. 2, folds 2, 3, and 4 that are normally present on tissue, and lesions 10, 20, and 30 are present on the surface of tissues (e.g., stomach or large intestine). The lesion 10 is a recessed early lesion that is depressed slightly, the lesion 20 is an elevated early lesion that protrudes slightly, and the lesion 30 is an early lesion in which the mucosal surface has become irregular. Note that a concavity-convexity part similar (e.g., in dimensions) to a concavity-convexity lesion is also observed in a normal area (see the concavity-convexity parts situated around the lesion 10, and the concavity-convexity parts situated on the right side of the fold 4). Since the method according to the first embodiment is intended to acquire the extracted concavity-convexity information that is useful for detecting a lesion or the like instead of detecting a lesion, the method according to the first embodiment does not distinguishes a concavity-convexity part included in a lesion from a concavity-convexity part included in a normal area.

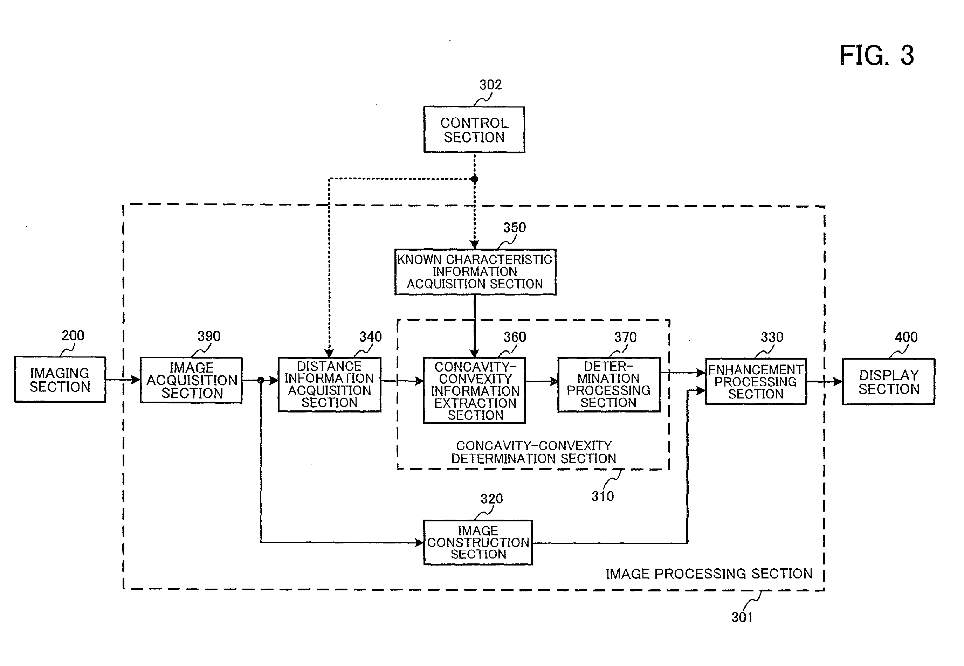

The details of the image processing section 301 are described below with reference to FIG. 3. The image processing section 301 includes an image acquisition section 390, an image construction section 320, a distance information acquisition section 340 (distance map calculation section), a known characteristic information acquisition section 350, a concavity-convexity determination section 310, and an enhancement processing section 330, and the concavity-convexity determination section 310 includes a concavity-convexity information extraction section 360 and a determination processing section 370.

The stereo image (left image and right image) output from the image sensors 206 and 207 included in the imaging section 200 is acquired by the image acquisition section 390, and the acquired stereo image is input to the image construction section 320 and the distance information acquisition section 340. The image construction section 320 performs given image processing (e.g., OB process, gain process, and $\gamma$ process) on the captured stereo image to generate an image that can be output to the display section 400. The resulting image is output to the enhancement processing section 330.

The distance information acquisition section 340 performs a matching calculation process on the left image (reference image) and a local area of the right image along an epipolar line that passes through the attention pixel positioned at the center of a local area of the left image to calculate the position at which the maximum correlation is obtained as a parallax. The distance information acquisition section 340 transforms the calculated parallax into the distance in the Z-direction to acquire distance information (distance map in a narrow sense). The acquired distance information is output to the concavity-convexity information extraction section 360 included in the concavity-convexity determination section 310.

The known characteristic information acquisition section 350 acquires the known characteristic information from the control section 302 (or a storage section that is not illustrated in FIG. 3). Specifically, the known characteristic information acquisition section 350 acquires the size (i.e., dimensional information (e.g., width, height, or depth)) of the extraction target concavity-convexity part of tissue due to a lesion, the size (i.e., dimensional information (e.g., width, height, or depth)) of the lumen and the folds of the observation target part based on observation target part information, and the like as the known characteristic information. Note that the observation target part information is information that represents the observation target part that is determined based on the scope ID information that is input to the control section 302 from the memory 210. The observation target part information may also be included in the known characteristic information. For example, when the scope is an upper gastrointestinal scope, the observation target part is the gullet, the stomach, or the duodenum. When the scope is a lower gastrointestinal scope, the observation target part is the large intestine. Since the dimensional information about the extraction target concavity-convexity part and the dimensional information about the lumen and the folds of the observation target part differ corresponding to each part, the known characteristic information acquisition section 350 outputs information about a typical size of a lumen and folds acquired based on the observation target part information to the concavity-convexity information extraction section 360, for example. Note that the observation target part information need not necessarily be determined based on the scope ID information. For example, the user may select the observation target part information using a switch provided to the external I/F section 500.

The concavity-convexity information extraction section 360 determines an extraction process parameter based on the known characteristic information, and extracts the extracted concavity-convexity information (performs the extracted concavity-convexity information extraction process) based on the determined extraction process parameter.

The concavity-convexity information extraction section 360 performs a low-pass filtering process on the input distance information using a given size (N×N pixels) to extract rough distance information. The concavity-convexity information extraction section 360 adaptively determines the extraction process parameter based on the extracted rough distance information. The details of the extraction process parameter are described later. The extraction process parameter may be the morphological kernel size (i.e., the size of a structural element) that is adapted to the distance information at the plane position orthogonal to the distance information of the distance map, a low-pass filter that is adapted to the distance information at the plane position, or a high-pass filter that is adapted to the plane position, for example. Specifically, the extraction process parameter is change information that changes an adaptive nonlinear or linear low-pass filter or high-pass filter corresponding to the distance information.

The concavity-convexity information extraction section 360 performs the extraction process based on the determined extraction process parameter to extract only the concavity-convexity parts of the object having the desired size. The determination processing section 370 links the extracted concavity-convexity parts to the captured image. The extracted concavity-convexity information refers to the information illustrated in FIG. 4C or FIG. 4E (described later), for example. It is considered that a given process may be required to link the extracted concavity-convexity information to the captured image. For example, when the extracted concavity-convexity information is acquired as a concavity-convexity image having a size that is a multiple of that of the captured image, the determination processing section 370 performs a scaling process or the like on the concavity-convexity image in order to transform the position of the concavity-convexity part within the concavity-convexity image into the position within the captured image. When the concavity-convexity information extraction section 360 acquires the extracted concavity-convexity information (concavity-convexity image) having the same size as that of the image output from the image construction section 320, the determination processing section 370 may be omitted, and the extracted concavity-convexity information may be output directly to the enhancement processing section 330.

The enhancement processing section 330 performs the desired enhancement process (e.g., a luminance enhancement process or a color (hue/chroma) enhancement process) corresponding to the specified concavity-convexity part on the captured image (e.g., the left image output from the image construction section 320 that is used as the reference image when calculating the parallax), and outputs only the processed left image to the display section 400. Specifically, the enhancement processing section 330 does not output a stereo image (three-dimensional image), and the display section 400 displays a 2D image. Note that the display image is not limited thereto. For example, the enhancement processing section 330 may output an enhanced stereo image. Alternatively, the enhancement processing section 330 may output both an enhanced 2D image and a stereo image that is not enhanced so that the images can be selectively displayed.

The details of the extraction process parameter determination process performed by the concavity-convexity information extraction section 360 are described below with reference to FIGS. 4A to 4F. In FIGS. 4A to 4F, the extraction process parameter is the diameter of a structural element (sphere) used for an opening process and a closing process (morphological process). FIG. 4A is a view schematically illustrating the surface of the object (tissue) and the vertical cross section of the imaging section 200. The folds 2, 3, and 4 present on the surface of the tissue are gastric folds, for example. The early lesions 10, 20, and 30 are present on the surface of the tissue.

The extraction process parameter determination process performed by the concavity-convexity information extraction section 360 is intended to determine the extraction process parameter for extracting only the early lesions 10, 20, and 30 from the surface of the tissue without extracting the folds 2, 3, and 4 from the surface of the tissue.

In order to determine such an extraction process parameter, it is necessary to use the size (i.e., dimensional information (e.g., width, height, or depth)) of the extraction target concavity-convexity part of tissue due to a lesion, and the size (i.e., dimensional information (e.g., width, height, or depth)) of the lumen and the folds of the observation target part based on the observation target part information (that are acquired from the control section 302).

It is possible to extract only the concavity-convexity parts having specific dimensions by determining the diameter of the sphere (with which the surface of the tissue is traced during the opening process and the closing process) using the above information. The diameter of the sphere is set to be smaller than the size of the lumen and the folds of the observation target part based on the observation target part information, and larger than the size of the extraction target concavity-convexity part of tissue due to a lesion. It is desirable to set the diameter of the sphere to be equal to or smaller than half of the size of the folds, and equal to or larger than the size of the extraction target concavity-convexity part of tissue due to a lesion. FIGS. 4A to 4F illustrate an example in which a sphere that satisfies the above conditions is used for the opening process and the closing process.

FIG. 4B illustrates the surface of the tissue after the closing process has been performed. As illustrated in FIG. 4B, information in which the concavities among the concavity-convexity parts having the extraction target dimensions are filled while maintaining the change in distance due to the wall surface of the tissue, and the structures such as the folds, is obtained by determining an appropriate extraction process parameter (i.e., the size of the structural element). Only the concavities on the surface of the tissue can be extracted (see FIG. 4C) by calculating the difference between information obtained by the closing process and the original surface of the tissue (see FIG. 4A).

FIG. 4D illustrates the surface of the tissue after the opening process has been performed. As illustrated in FIG. 4D, information in which the convexities among the concavity-convexity parts having the extraction target dimensions are removed, is obtained by the opening process. Only the convexities on the surface of the tissue can be extracted (see FIG. 4E) by calculating the difference between information obtained by the opening process and the original surface of the tissue.

The opening process and the closing process may be performed on the surface of the tissue using a sphere having an identical size. However, since the stereo image is characterized in that the area of the image formed on the image sensor decreases as the distance represented by the distance information increases, the diameter of the sphere may be increased when the distance represented by the distance information is short, and may be decreased when the distance represented by the distance information is long, in order to extract a concavity-convexity part having the desired size.

FIG. 4F illustrates an example in which the diameter of the sphere is changed with respect to the average distance information when performing the opening process and the closing process on the distance map. Specifically, it is necessary to correct the actual size of the surface of the tissue using the optical magnification to agree with the pixel pitch of the image formed on the image sensor in order to extract the desired concavity-convexity part with respect to the distance map. Therefore, the concavity-convexity information extraction section 360 may acquire the optical magnification of the imaging section 200 determined based on the scope ID information from the memory 210, for example.

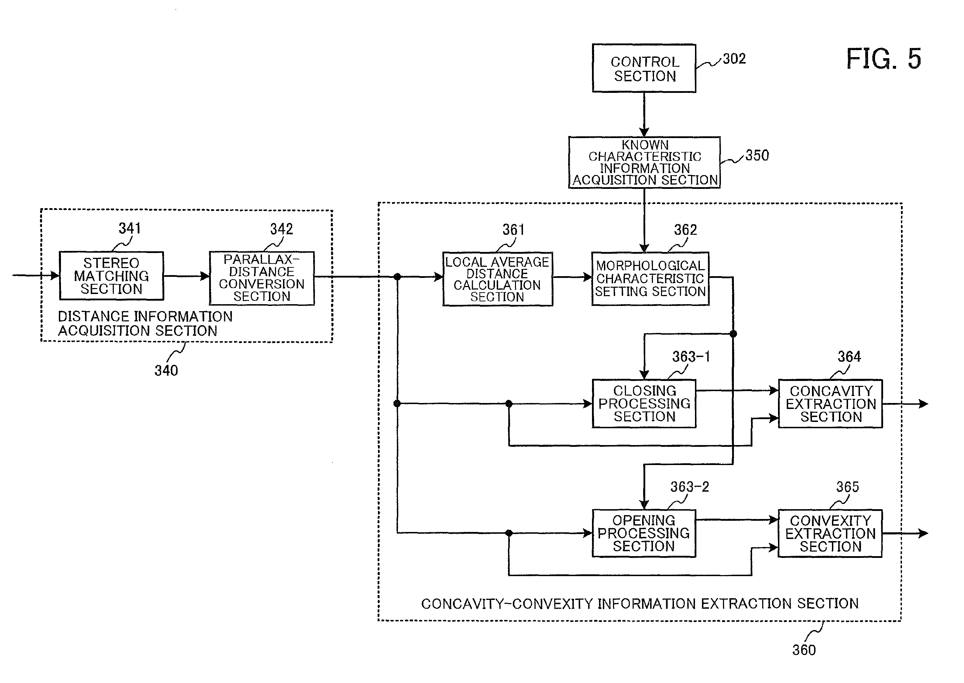

FIG. 5 illustrates a detailed block diagram of the distance information acquisition section 340, the known characteristic information acquisition section 350, and the concavity-convexity information extraction section 360. The distance information acquisition section 340 includes a stereo matching section 341 and a parallax-distance conversion section 342. The concavity-convexity information extraction section 360 includes a local average distance calculation section 361, a morphological characteristic setting section 362, a closing processing section 363-1, an opening processing section 363-2, a concavity extraction section 364, and a convexity extraction section 365.

The stereo image output from the imaging section 200 is input to the stereo matching section 341, and the stereo matching section 341 performs a block matching process on the left image (reference image) and the right image with respect to the processing target pixel and its peripheral area (i.e., a block having a given size) using an epipolar line to calculate parallax information. The parallax-distance conversion section 342 converts the calculated parallax information into the distance information. This conversion process includes a process that corrects the optical magnification of the imaging section 200.

The parallax-distance conversion section 342 outputs the distance information to the local average distance calculation section 361 as the distance map (having the same pixel size as that of the stereo image in a narrow sense). The local average distance calculation section 361 performs an average value calculation process (e.g., $3 \times 3$ pixels) on the input distance map to calculate the average distance in a local area. The calculated average distance is input to the morphological characteristic setting section 362, and the morphological characteristic setting section 362 determines the diameter of the sphere (extraction process parameter) used for the opening process and the closing process using the size (i.e., dimensional information (e.g., width, height, or depth)) of the extraction target concavity-convexity part of tissue due to a lesion, and the size (i.e., dimensional information (e.g., width, height, or depth)) of the lumen and the folds of the observation target part based on the observation target part information (that are acquired from the control section 302).

Information about the diameter of the sphere thus determined is input to the closing processing section 363-1 and the opening processing section 363-2 as a diameter map having the same number of pixels as that of the distance map. The closing processing section 363-1 and the opening processing section 363-2 respectively perform the closing process and the opening process while changing the diameter of the sphere on a pixel basis using the diameter map. The processing results of the closing processing section 363-1 are output to the concavity extraction section 364. The processing results of the opening processing section 363-2 are output to the convexity extraction section 365.

The distance map before the closing process and the distance map after the closing process are input to the concavity extraction section 364, and the distance map after the closing process is subtracted from the distance map before the closing process to output a concavity image in which only the desired concavities are extracted. The distance map before the opening process and the distance map after the opening process are input to the convexity extraction section 365, and the distance map after the opening process is subtracted from the distance map before the opening process to output a convexity image in which only the desired convexities are extracted.

According to the first embodiment, since the extraction target concavity-convexity part of tissue due to a lesion can be extracted with high accuracy without being affected by the shape of the folds and the lumen of the observation target part, it is possible to selectively enhance the concavity-convexity part due to a lesion within the display image, for example.

According to the first embodiment, the image processing device includes the image acquisition section 390 that acquires a captured image that includes an image of an object, the captured image being an image captured by the imaging section 200, the distance information acquisition section 340 that acquires the distance information based on the distance from the imaging section 200 to the object when the imaging section 200 captured the captured image, the known characteristic information acquisition section 350 that acquires the known characteristic information, the known characteristic information being information that represents the known characteristics relating to the structure of the object, and the concavity-convexity determination section 310 that performs the concavity-convexity determination process that specifies a concavity-convexity part of the object that agrees with the characteristics specified by the known characteristic information, from the object captured within the captured image, based on the distance information and the known characteristic information (see FIGS. 1 and 3).

The term "distance information" used herein refers to information that is acquired based on the distance from the imaging section 200 to the object. For example, when implementing triangulation using a stereo optical system (see above), the distance with respect to an arbitrary point in a plane that connects two lenses (i.e., the objective lenses 204 and 205 illustrated in FIG. 2) that produce a parallax may be used as the distance information. When using the Time-of-Flight method described later in connection with the second embodiment and the like, the distance with respect to each pixel position in the plane of the image sensor is acquired as the distance information, for example. In such a case, the distance measurement reference point is set to the imaging section 200. Note that the distance measurement reference point may be set to an arbitrary position other than the imaging section 200, such as an arbitrary position within the three-dimensional space that includes the imaging section and the object. The distance information acquired using such a reference point is also intended to be included within the term "distance information".

The distance from the imaging section 200 to the object may be the distance from the imaging section 200 to the object in the depth direction, for example. For example, the distance from the imaging section 200 to the object may be the distance from the imaging section 200 to the object in the direction of the optical axis of the imaging section 200. When the viewpoint is set in the direction perpendicular to the optical axis (see FIG. 4A), the distance from the imaging section 200 to the object may be the distance from the imaging section 200 to the object observed from the viewpoint (e.g., the distance from the imaging section 200 to the object in the vertical direction (see the arrow) in the example illustrated in FIG. 4A).

For example, the distance information acquisition section 340 may transform the coordinates of each corresponding point in a first coordinate system in which a first reference point of the imaging section 200 is the origin, into the coordinates of each corresponding point in a second coordinate system in which a second reference point within the three-dimensional space is the origin, using a known coordinate transformation process, and measure the distance based on the coordinates obtained by transformation. In this case, the distance from the second reference point to each corresponding point in the second coordinate system is identical with the distance from the first reference point to each corresponding point in the first coordinate system (i.e., the distance from the imaging section to each corresponding point).

The distance information acquisition section 340 may set a virtual reference point at a position that can maintain a relationship similar to the relationship between the distance values of the pixels on the distance map acquired when setting the reference point to the imaging section 200, to acquire the distance information based on the distance from the imaging section 200 to each corresponding point. For example, when the actual distances from the imaging section 200 to three corresponding points are respectively "3", "4", and "5", the distance information acquisition section 340 may acquire distance information "1.5", "2", and "2.5" respectively obtained by halving the actual distances "3", "4", and "5" while maintaining the relationship between the distance values of the pixels. In this case, the concavity-convexity information acquisition section 360 uses a different extraction process parameter as compared with the case of setting the reference point to the imaging section 200. Specifically, since it is necessary to use the distance information when determining the extraction process parameter, the extraction process parameter is determined in a different way when the distance measurement reference point has changed (i.e., when the distance information is represented in a different way). For example, when extracting the extracted concavity-convexity information using the morphological process (see above), the size of the structural element (e.g., the diameter of a sphere) used for the extraction process is adjusted, and the concavity-convexity part extraction process is performed using the structural element that has been adjusted in size.

According to this configuration, since it is possible to specify a concavity-convexity part having given characteristics from the object captured within the captured image based on the distance information and the known characteristic information, it is possible to accurately detect the concavity-convexity part of the object (i.e., the concavity-convexity part on the surface of the object in a narrow sense). Note that the concavity-convexity part is not limited to the concavity-convexity part on the surface of the object. Since it is unnecessary to effect a change in the object (e.g., by spraying a dye), it is unnecessary to take account of a decrease in visibility of an object other than the enhancement target, and it is possible to use less invasive procedure when performing the process on tissue, for example.

The concavity-convexity determination section 310 may include the concavity-convexity information extraction section 360 that extracts the extracted concavity-convexity information that represents the concavity-convexity part of the object that agrees with the characteristics specified by the known characteristic information from the distance information, based on the distance information and the known characteristic information. The concavity-convexity determination section 310 may perform the concavity-convexity determination process based on the extracted concavity-convexity information.

This makes it possible to extract the extracted concavity-convexity information (e.g., the information illustrated in FIG. 4C or 4E) from the distance information (e.g., the information illustrated in FIG. 4A), and specify the concavity-convexity part within the captured image using the extracted concavity-convexity information. Specifically, it is possible to specify the area of the captured object in which the desired concavity-convexity part is situated, by acquiring the information about the concavity-convexity part having the desired characteristics using the distance information including the three-dimensional information about the object, and linking the acquired information to the captured image. Note that the extracted concavity-convexity information in a narrow sense may represent a concavity-convexity image in which the number of pixels corresponds to the distance map or the image generated by the image construction section 320 (e.g., the same number of pixels as that of the distance map or the image generated by the image construction section 320), and each pixel value is a value that corresponds to a convexity or a concavity. For example, a value that corresponds to a convexity may be a positive value, a value that corresponds to a concavity may be a negative value, and the absolute value of each value may increase as the height of the convexity increases, or the depth of the concavity increases. Note that the extracted concavity-convexity information is not limited to the concavity-convexity image, but may be another type of information.

The concavity-convexity information extraction section 360 may determine the extraction process parameter based on the known characteristic information, and extract the concavity-convexity part of the object as the extracted concavity-convexity information based on the determined extraction process parameter.

This makes it possible to perform the extracted concavity-convexity information extraction process (e.g., separation process) using the extraction process parameter determined based on the known characteristic information. The extraction process may be performed using the morphological process (see above), a filtering process (described later), or the like. In order to accurately extract the extracted concavity-convexity information, it is necessary to perform a control process that extracts information about the desired concavity-convexity part from information about various structures included in the distance information while excluding other structures (e.g., the original structures of tissue such as folds). The above control process is implemented by setting the extraction process parameter based on the known characteristic information.

The known characteristic information acquisition section 350 may acquire type information and concavity-convexity characteristic information as the known characteristic information, the type information being information that represents the type of the object, and the concavity-convexity characteristic information being information about the concavity-convexity part of the object that is linked to the type information. The concavity-convexity information extraction section 360 may determine the extraction process parameter based on the type information and the concavity-convexity characteristic information, and extract the concavity-convexity part of the object as the extracted concavity-convexity information based on the determined extraction process parameter.

The term "type information" used herein refers to information that specifies the type of the object. For example, when applying the image processing device to an industrial endoscope, the type information may be information that specifies the observation target device or the like. The type information may be information that specifies the type within a narrower range. For example, the type information may be information that specifies the observation target pipe among a plurality of pipes that are included in a device and differ in thickness. The term "concavity-convexity characteristic information" used herein refers to information that specifies the characteristics of the concavity-convexity part of the object that is to be extracted from the distance information. Specifically, the concavity-convexity characteristic information includes at least one of information that represents the characteristics of the exclusion target concavity-convexity part among the concavity-convexity parts included in the distance information, and information that represents the characteristics of the extraction target concavity-convexity part among the concavity-convexity parts included in the distance information.

This makes it possible to determine the extraction process parameter using the type information and the concavity-convexity characteristic information as the known characteristic information. The dimensions and the like of the extraction target concavity-convexity part differ corresponding to the type of the observation target (see the above example relating to the thickness of the pipe). Therefore, the image processing device according to the first embodiment stores a plurality of pieces of concavity-convexity characteristic information corresponding to each piece of type information, and determines an appropriate extraction process parameter by selecting appropriate concavity-convexity characteristic information corresponding to the acquired type information. Note that the concavity-convexity characteristic information may be one piece of information (reference information), and may be converted corresponding to the type information.

The captured image may be *an in vivo* image that is obtained by capturing inside of a living body, and the known characteristic information acquisition section 350 may acquire part information and concavity-convexity characteristic information as the known characteristic information, the part information being information that represents a part of the living body to which the object corresponds, and the concavity-convexity characteristic information being information about the concavity-convexity part of the living body. The concavity-convexity information extraction section 360 may determine the extraction process parameter based on the part information and the concavity-convexity characteristic information, and extract the concavity-convexity part of the object as the extracted concavity-convexity information based on the determined extraction process parameter.

This makes it possible to acquire the part information about a part (object) within an *in vivo* image as the known characteristic information when applying the method according to the first embodiment to *an in vivo* image (e.g., when applying the image processing device according to the first embodiment to a medical endoscope apparatus). When applying the method according to the first embodiment to *an in vivo* image, it is considered that a concavity-convexity structure useful for detecting an early lesion or the like is extracted as the extracted concavity-convexity information. However, the characteristics (e.g., dimensional information) of a concavity-convexity part specific to an early lesion may differ corresponding to each part. The exclusion target structure (e.g., fold) of tissue necessarily differs corresponding to each part. Therefore, it is necessary to perform an appropriate process corresponding to each part when applying the method according to the first embodiment to tissue. In the first embodiment, such a process is performed based on the part information.

In this case, various methods may be used. For example, a storage section (not illustrated in the drawings) may store first concavity-convexity characteristic information to Nth concavity-convexity characteristic information that respectively correspond to first to Nth parts. When it has been specified that the object corresponds to the kth part based on the part information, the concavity-convexity information extraction section 360 may determine the extraction process parameter using the kth concavity-convexity characteristic information among the first concavity-convexity characteristic information to the Nth concavity-convexity characteristic information. Alternatively, a storage section (not illustrated in the drawings) may store reference concavity-convexity characteristic information as the concavity-convexity characteristic information, and the concavity-convexity information extraction section 360 may perform a conversion process on the reference concavity-convexity characteristic information based on the part information, and determine the extraction process parameter using the concavity-convexity characteristic information after the conversion process.

The concavity-convexity information extraction section 360 may determine the size of the structural element used for the opening process and the closing process as the extraction process parameter based on the known characteristic information, and perform the opening process and the closing process using the structural element having the determined size to extract the concavity-convexity part of the object as the extracted concavity-convexity information.

This makes it possible to extract the extracted concavity-convexity information based on the opening process and the closing process (morphological process in a broad sense) (see FIGS. 4A to 4F). In this case, the extraction process parameter is the size of the structural element used for the opening process and the closing process. In the example illustrated in FIG. 4A, the structural element is a sphere, and the extraction process parameter is a parameter that represents the diameter of the sphere, for example. Specifically, the size of the structural element is determined so that the exclusion target shape (e.g., fold) is not deformed (i.e., the sphere moves to follow the exclusion target shape) when the process using the structural element is performed on the exclusion target shape (when the sphere is moved on the surface in FIG. 4A). The size of the structural element may be determined so that the extraction target concavity-convexity part (extracted concavity-convexity information) is removed (i.e., the sphere does not enter the concavity or the convexity) when the process using the structural element is performed on the extraction target concavity-convexity part. Since the morphological process is a well-known process, detailed description thereof is omitted.

The concavity-convexity information extraction section 360 may decrease the size of the structural element used as the extraction process parameter as the value represented by the distance information that corresponds to the processing target pixel of the opening process and the closing process increases.

This makes it possible to link the actual size to the size within the image. Since the known characteristic information represents the known characteristics relating to the structure of the object., it is considered that the known characteristic information is represented by the actual size (e.g., $\mu$m or mm) in the actual space. However, since the distance from the imaging section 200 to the object varies as illustrated in FIG. 4A and the like, an object situated closer to the imaging section 200 is observed to have a large size as compared with an object situated away from the imaging section 200, even when these objects have an identical actual size (e.g., grooves having an identical width). Therefore, it is difficult to extract the concavity-convexity part that agrees with the characteristics specified by the known characteristic information unless a conversion process is performed on the actual size and the apparent size represented by the distance information (e.g., a size determined on a pixel basis when using a distance map in which the distance information is on a pixel basis). Therefore, the conversion process is performed by changing the extraction process parameter corresponding to the value represented by the distance information. Specifically, since the extraction target concavity-convexity part is observed to have a smaller size as the distance from the imaging section 200 increases, the size of the structural element is decreased. Since the relationship between the actual size and the size within the image changes depending on the imaging magnification of the imaging section 200, it is desirable that the concavity-convexity information extraction section 360 acquire information about the imaging magnification from the imaging section 200, and also perform the conversion process using the imaging magnification.

The object may include a global three-dimensional structure, and a local concavity-convexity structure that is more local than the global three-dimensional structure, and the concavity-convexity information extraction section 360 may extract the concavity-convexity part of the object that is selected from the global three-dimensional structure and the local concavity-convexity structure included in the object, and agrees with the characteristics specified by the known characteristic information, as the extracted concavity-convexity information.

This makes it possible to determine whether to extract either the global structure or the local structure when the object includes the global structure and the local structure, and extract information about the determined structure as the extracted concavity-convexity information.

The captured image may be *an in vivo* image that is obtained by capturing inside of a living body, the object may include a global three-dimensional structure that is a lumen structure inside the living body, and a local concavity-convexity structure that is formed on the lumen structure, and is more local than the global three-dimensional structure, and the concavity-convexity information extraction section 360 may extract the concavity-convexity part of the object that is selected from the global three-dimensional structure and the local concavity-convexity structure included in the object, and agrees with the characteristics specified by the known characteristic information, as the extracted concavity-convexity information.

This makes it possible to implement a process that extracts the concavity-convexity part from the global three-dimensional structure (i.e., a structure having a low spatial frequency as compared with the concavity-convexity part) and the concavity-convexity part included in the distance information when applying the method according to the first embodiment to *an in vivo* image. When applying the method according to the first embodiment to an *in vivo* image, the extraction target is a concavity-convexity part useful for finding an early lesion. Specifically, the three-dimensional structure (e.g., folds and a structure based on the curvature of a wall surface) of tissue can be excluded from the extraction target, and the concavity-convexity information extraction section 360 extracts only the extraction target concavity-convexity part. In this case, since the global structure (i.e., a structure having a low spatial frequency) is excluded from the extraction target, and the local structure (i.e., a structure having a high spatial frequency) is determined to be the extraction target, a process that sets the intermediate spatial frequency to be the boundary (i.e., the extraction process parameter in a narrow sense) is performed, for example.

The distance information acquisition section 340 may acquire the distance map as the distance information, the distance map being a map in which information about the distance from the imaging section to the object captured at each pixel of the acquired captured image being linked to each pixel of the acquired captured image.

The term "distance map" used herein is a narrower concept of the distance information, and refers to a map in which the distance (depth) to the object in the Z-axis direction (i.e., the direction of the optical axis of the imaging section 200) is specified for each point (e.g., each pixel) in the XY plane, for example.

This makes it possible to acquire the distance map as the distance information. In this case, it is possible to easily link the distance information, the extracted concavity-convexity information that is extracted from the distance information, and the image of the object obtained by the image construction process or the like (i.e., the image acquired by the image construction section 320) on a pixel basis. Therefore, it is possible to easily determine the position of the extraction target concavity-convexity part within the image of the object, and easily determine the enhancement target pixel when performing the enhancement process on the concavity-convexity part using the enhancement processing section 330, for example.

The imaging section 200 may include a plurality of viewpoints, the image acquisition section 390 may acquire a plurality of captured images that respectively correspond to the plurality of viewpoints, and the distance information acquisition section 340 may acquire the distance information based on parallax information obtained from the plurality of captured images acquired by the image acquisition section 390.

This makes it possible to acquire the distance information based on the parallax information obtained from the plurality of captured images that respectively correspond to the plurality of viewpoints. The parallax information acquisition process, the process that converts the parallax information into the distance information, and the like are widely known as a stereo matching process, and detailed description thereof is omitted. In this case, since a known image sensor such as a Bayer array single-chip image sensor can be used as the image sensor (the image sensors 206 and 207 in FIG. 2), implementation is easy. Since such an image sensor has been reduced in size, for example, it is possible to reduce the size of the imaging section 200, although image sensors and optical systems in a number corresponding to the number of viewpoints are required, and it is possible to apply such a configuration to various fields (e.g., endoscope apparatus).

The first embodiment may also be applied to an electronic device that includes the image processing device.

This makes it possible to implement an electronic device that detects a concavity-convexity part using the method according to the first embodiment. The electronic device may include the imaging section 200 and hardware for generating the distance information (e.g., a stereo optical system (see above), a laser light source 105 that utilizes the Time-of-Flight method, or a range sensor 214), may include one of the imaging section 200 and hardware for generating the distance information, or may not include the imaging section 200 and hardware for generating the distance information. The electronic device according to the first embodiment may be a device (e.g., PC or server) that acquires information from a satellite, for example. In this case, the distance from the satellite to Mt. Fuji may be measured by causing the satellite to emit a laser beam, and the electronic device according to the first embodiment may acquire an image of Mt. Fuji and the distance information from the satellite through a network. The electronic device may acquire geometrical information about the caldera (concavity) formed at the top of Mt. Fuji from a storage section as the known characteristic information, and determine the caldera formed at the top of Mt. Fuji within the image based on the known characteristic information.

The first embodiment may also be applied to an endoscope apparatus (see FIG. 2) that includes the image processing device.

This makes it possible to implement an endoscope apparatus that detects a concavity-convexity part using the method according to the first embodiment. It has been known that a minute concavity-convexity part of tissue is useful for finding an early lesion. However, the concavity-convexity part detection accuracy implemented by a known method may be insufficient, or an invasive method that sprays a dye has been used instead of image processing, for example. Since the method according to the first embodiment can accurately detect a concavity-convexity part by image processing, the method according to the first embodiment is useful in the medical field and the like. Since the method according to the first embodiment detects an concavity-convexity part having characteristics similar to those of an concavity-convexity part observed in an early lesion or the like, the method according to the first embodiment detects not only the concavity-convexity parts in the lesions 10, 20, and 30 illustrated in FIG. 2, but also the concavity-convexity parts in the normal area. Specifically, although the extracted concavity-convexity information to be output is useful for finding an early lesion, the method according to the first embodiment is not intended to provide an early lesion detection method.

Note that part or most of the process performed by the image processing device and the like according to the first embodiment may be implemented by a program. In this case, the image processing device and the like according to the first embodiment are implemented by causing a processor (e.g., CPU) to execute a program. More specifically, a program stored in an information storage device is read, and executed by a processor (e.g., CPU). The information storage device (computer-readable device) stores a program, data, and the like. The function of the information storage device may be implemented by an optical disk (e.g., DVD or CD), a hard disk drive (HDD), a memory (e.g., memory card or ROM), or the like. The processor (e.g., CPU) performs various processes according to the first embodiment based on the program (data) stored in the information storage device. Specifically, a program that causes a computer (i.e., a device that includes an operation section, a processing section, a storage section, and an output section) to function as each section according to the first embodiment (i.e., a program that causes a computer to execute the process implemented by each section) is stored in the information storage device.

3. Second embodiment

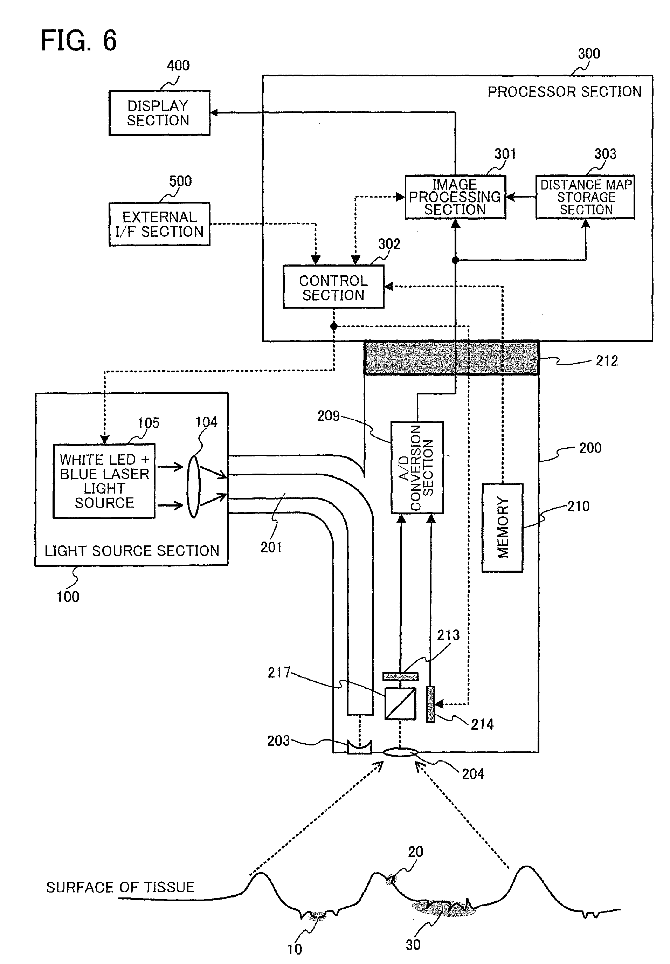

FIG. 6 is a functional block diagram illustrating an endoscope apparatus according to the second embodiment. The endoscope apparatus according to the second embodiment includes a light source section 100, an imaging section 200, a processor section 300, a display section 400, and an external I/F section 500.

The light source section 100 includes a white LED, a blue laser light source 105, and a condenser lens 104 that focuses light obtained by synthesizing light emitted from the white LED and light emitted from the blue laser on the incident end face of a light guide fiber 201. The white LED and the blue laser light source 105 are controlled by a control section 302 in a pulsed manner. The blue laser emits light having a wavelength shorter than that of light emitted from the white LED, for example.

The imaging section 200 is formed to be elongated and flexible so that the imaging section 200 can be inserted into a body cavity (e.g., stomach or large intestine), for example. The imaging section 200 includes the light guide fiber 201 that guides the light focused by the light source section 100, an illumination lens 203 that diffuses the light that has been guided by the light guide fiber 201, and applies the diffused light to the observation target, an objective lens 204 that focuses the reflected light from the observation target, a dichroic prism 217 that reflects only the focused light having the wavelength of the blue laser light, and allows the focused light having a wavelength other than the wavelength of the blue laser light to pass through, a range sensor 214 that utilizes the Time-of-Flight method, and detects the time from the blue laser light emission start time to the capture (imaging) start time, and an image sensor 213 that detects the light emitted from the white LED. The imaging section 200 also includes an A/D conversion section 209 that converts photoelectrically-converted analog signals output from the image sensor 213, and analog signals (distance information) output from the range sensor 214 into digital signals, a memory 210 that stores scope ID information and specific information (including a production variation) about the imaging section 200, and a connector 212 for removably connecting the imaging section 200 and the processor section 300. The image sensor 213 is a primary-color single-chip image sensor (Bayer array), and may be implemented by a CCD sensor, a CMOS sensor, or the like.

The image output from the image sensor 213 is converted into digital signals by the A/D conversion section 209, and output to an image processing section 301, and the distance information output from the range sensor 214 is converted into digital signals by the A/D conversion section 209, and output to a distance map storage section 303. The memory 210 is connected to the control section 302, and the scope ID information and the specific information (including a production variation) are transmitted to the control section 302.

The processor section 300 includes the image processing section 301, the control section 302, and the distance map storage section 303.

The display section 400 is a display device (e.g., CRT or liquid crystal monitor) that can display a movie (moving image).

The external I/F section 500 is an interface that allows the user to input information to the endoscope apparatus, for example. The external I/F section 500 includes a power switch (power ON/OFF switch), a shutter button for starting an imaging operation, a mode (e.g., imaging mode) switch button (e.g., a switch for selectively performing an enhancement process on a concavity-convexity part present on the surface of tissue), and the like. The external I/F section 500 outputs the input information to the control section 302.

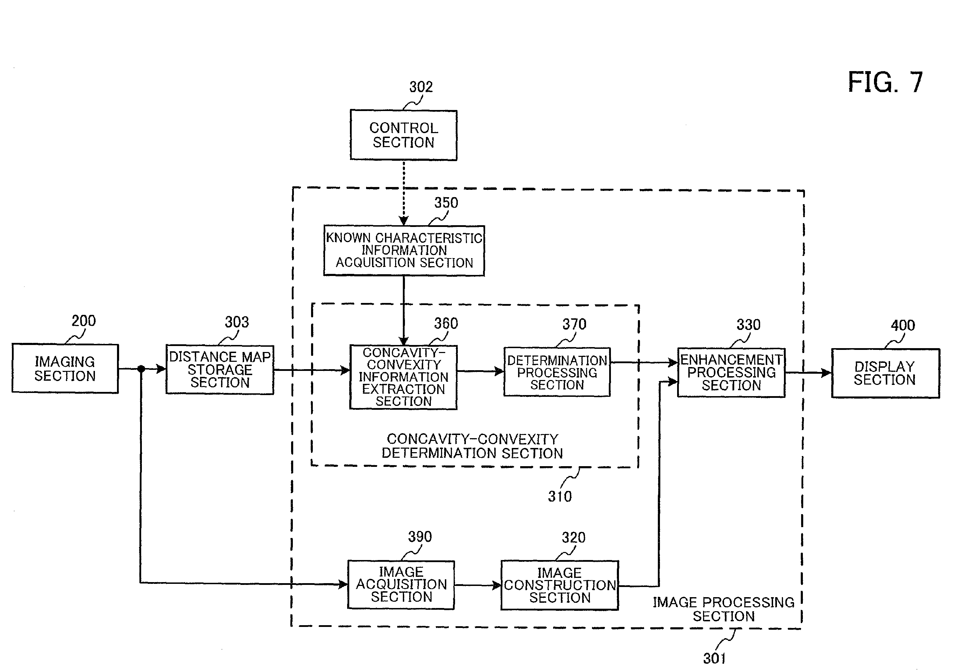

The details of the image processing section 301 are described below with reference to FIG. 7. The image processing section 301 includes an image acquisition section 390, an image construction section 320, a known characteristic information acquisition section 350, a concavity-convexity determination section 310, and an enhancement processing section 330, and the concavity-convexity determination section 310 includes a concavity-convexity information extraction section 360 and a determination processing section 370.

The image output from the image sensor 213 included in the imaging section 200 is acquired by the image acquisition section 390, and the acquired image is input to the image construction section 320. The distance information output from the range sensor 214 is input to the distance map storage section 303. The image construction section 320 performs given image processing (e.g., OB process, gain process, and γ process) on the captured image to generate an image that can be output to the display section 400. The resulting image is output to the enhancement processing section 330.

The distance map (having the same number of pixels as that of the image sensor 213) output from the range sensor 214 that is stored in the distance map storage section 303 is output to the concavity-convexity information extraction section 360.

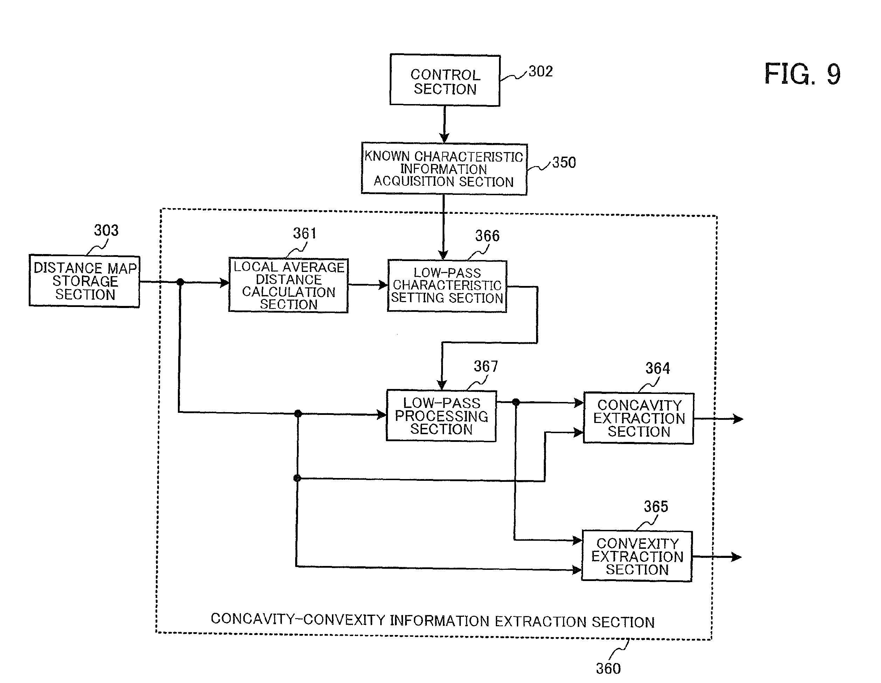

The concavity-convexity information extraction section 360 according to the second embodiment is described below with reference to FIG. 9. The concavity-convexity information extraction section 360 includes a local average distance calculation section 361, a low-pass characteristic setting section 366, a low-pass processing section 367, a concavity extraction section 364, and a convexity extraction section 365.

The concavity-convexity information extraction section 360 according to the second embodiment performs a process similar to the morphological process described above in connection with the first embodiment by changing the frequency characteristics of a low-pass filter using the local average distance. The low-pass filter may be a linear Gaussian filter, or may be a nonlinear bilateral filter. Specifically, since the size of a concavity-convexity part due to a lesion within the image increases when the distance is short, and decreases when the distance is long, it is necessary to generate a reference plane required for extracting the desired concavity-convexity part by changing characteristics of the low-pass filter based on the distance information. Specifically, the extraction process parameter according to the second embodiment is a parameter that determines the characteristics (frequency characteristics in a narrow sense) of the low-pass filter.

The distance map output from the distance map storage section 303 is subjected to an average value calculation process (e.g., 3×3 pixels) (see the first embodiment) performed by the local average distance calculation section 361, and output to the low-pass characteristic setting section 366.

The size (i.e., dimensional information (e.g., width, height, or depth)) of the extraction target concavity-convexity part of tissue due to a lesion, the size (i.e., dimensional information (e.g., width, height, or depth)) of the lumen and the folds of the observation target part based on observation target part information, and the like are input to the low-pass characteristic setting section 366 from the known characteristic information acquisition section 350 as the known characteristic information. The optical magnification of the imaging section 200, and the local average distance are also input to the low-pass characteristic setting section 366. The size of the distance map (Z-direction) and the size of the object corresponding to the coordinate system (pixel pitch) orthogonal to the Z-direction are caused to coincide with each other, and the characteristics of the low-pass filter are determined so that the extraction target concavity-convexity part of tissue due to a lesion can be smoothed, and the structure of the lumen and the folds of the observation target part can be maintained.

The low-pass filter may be a known Gaussian filter or bilateral filter. The characteristics of the low-pass filter may be controlled using a parameter σ, and a σ map corresponding to each pixel of the distance map may be generated. When using a bilateral filter, the σ map may be generated using either or both of a luminance difference parameter σ and a distance parameter σ. Note that the term "luminance" used herein in connection with the luminance difference parameter σ refers to the pixel value when the distance map is considered to be an image, and the luminance difference refers to the difference in distance in the Z-direction. The term "distance" used herein in connection with the distance parameter σ refers to the distance between the attention pixel and its peripheral pixel in the XY-direction. A Gaussian filter is represented by the following expression (1), and a bilateral filter is represented by the following expression (2).

$$f(x) = \frac{1}{N} \exp\left(-\frac{(x-x0)^2}{2\sigma^2}\right) \quad \cdots \cdots \cdots (1)$$

$$f(x) = \frac{1}{N} \exp\left(-\frac{(x-x0)^2}{2\sigma_c^2}\right) \times \exp\left(-\frac{(p(x)-p(x0))^2}{2\sigma_v^2}\right) \quad \cdots \cdots (2)$$

For example, a σ map subjected to a pixel thinning process may be generated, and output to the low-pass processing section 367. The low-pass processing section 367 applies the desired low-pass filter to the distance map using the distance map and the σ map.

The parameter σ that determines the characteristics of the low-pass filter is set to be larger than a value obtained by multiplying the pixel-to-pixel distance D1 of the distance map corresponding to the size of the extraction target concavity-convexity part by α (>1), and smaller than a value obtained by multiplying the pixel-to-pixel distance D2 of the distance map corresponding to the size of the lumen and the folds specific to the observation target part by β (<1). For example, the parameter σ may calculated by σ=(α*D1+β*D2)/2*Rσ.

Steeper sharp-cut characteristics may be set as the characteristics of the low-pass filter. In this case, the filter characteristics are controlled using a cut-off frequency fc instead of the parameter σ. The cut-off frequency fc may be set so that a frequency F1 in the cycle D1 does not pass through, and a frequency F2 in the cycle D2 does pass through. For example, the cut-off frequency fc may be set to fc=(F1+F2)/2*Rf.

Note that Rσ is a function of the local average distance. The output value increases as the local average distance decreases, and decreases as the local average distance increases. Rf is a function that is designed so that the output value decreases as the local average distance decreases, and increases as the local average distance increases.

The output from the low-pass processing section 367 and the distance map output from the distance map storage section 303 are input to the concavity extraction section 364. A concavity image can be output by extracting only a negative area obtained by subtracting the low-pass filtering results from the distance map that is not subjected to the low-pass filtering process. The output from the low-pass processing section 367 and the distance map output from the distance map storage section 303 are input to the convexity extraction section 365. A convexity image can be output by extracting only a positive area obtained by subtracting the low-pass filtering results from the distance map that is not subjected to the low-pass filtering process.

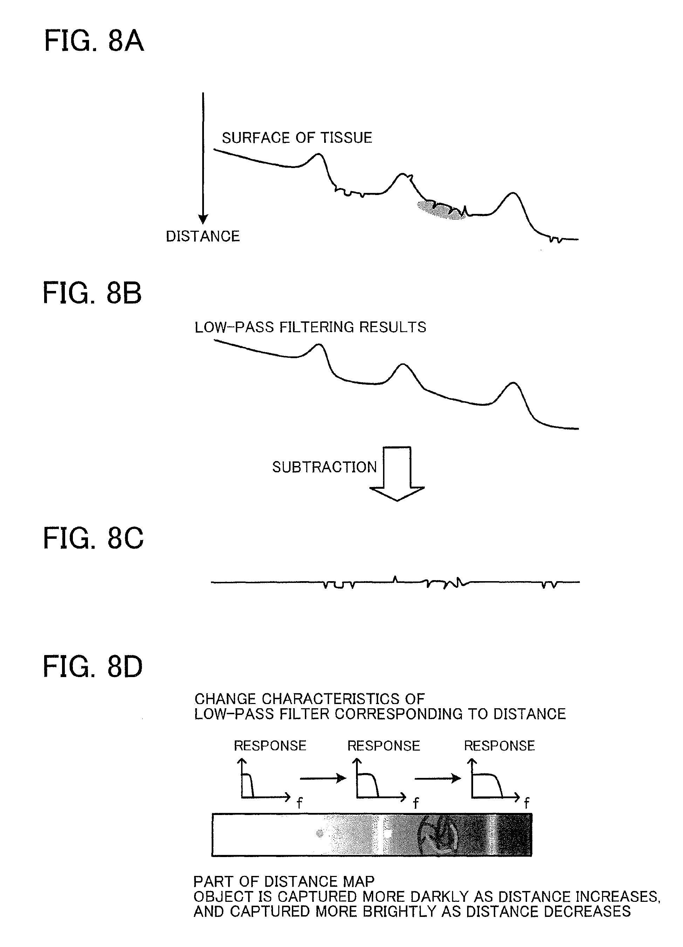

FIGS. 8A to 8D illustrate extraction of the desired concavity-convexity part due to a lesion using the low-pass filter. As illustrated in FIG. 8B, information in which the concavity-convexity parts having the extraction target dimensions are removed while maintaining the change in distance due to the wall surface of the tissue, and the structures such as the folds, is obtained by performing the filtering process using the low-pass filter on the distance map illustrated in FIG. 8A. Since the low-pass filtering results serve as a reference plane for extracting the desired concavity-convexity parts (see FIG. 8B) even if the opening process and the closing process described above in connection with the first embodiment are not performed, the concavity-convexity parts can be extracted (see FIG. 8C) by performing a subtraction process on the original distance map (see FIG. 8A). In the second embodiment, the characteristics of the low-pass filter are changed corresponding to the rough distance information in the same manner as in the first embodiment in which the size of the structural element is adaptively changed corresponding to the rough distance information. FIG. 8D illustrates an example in which the characteristics of the low-pass filter are changed corresponding to the rough distance information.

The subsequent process is the same as described above in connection with the first embodiment, and description thereof is omitted.

According to the second embodiment, since the range sensor that utilizes the Time-of-Flight method is provided, and the blue laser is used as the ranging light source, it is possible to extract the distance information that corresponds to the concavity-convexity parts present on the surface of tissue while suppressing a situation in which light enters the mucous membrane. This makes it possible to accurately extract only the extraction target concavity-convexity part of tissue due to a lesion without being affected by the shape of the folds and the lumen of the observation target part.

According to the second embodiment, the concavity-convexity information extraction section 360 determines the frequency characteristics of the filter used for the filtering process performed on the distance information as the extraction process parameter based on the known characteristic information, and perform the filtering process that utilizes the filter having the determined frequency characteristics to extract the concavity-convexity part of the object as the extracted concavity-convexity information.

This makes it possible to extract the extracted concavity-convexity information based on the filtering process (see FIGS. 8A to 8D). Although an example in which the filtering process utilizes the low-pass filter has been described above, the filtering process may utilize a high-pass filter (see the third embodiment) or a band-pass filter. In this case, the extraction process parameter is the characteristics (i.e., spatial frequency characteristics in a narrow sense) of the filter used for the filtering process. Specifically, the parameter $\sigma$ and the cut-off frequency are determined based on the frequency that corresponds to the exclusion target (e.g., fold) and the frequency that corresponds to the concavity-convexity part (see above).

The imaging section 200 may include a light source section that emits blue light (blue laser light source 105), and a ranging device (range sensor 214) that receives reflected blue light from the object (see FIG. 6), and the distance information acquisition section 340 may acquire the distance information based on time information about the time from the timing at which the blue light was emitted from the light source section to the timing at which the ranging device received the reflected light.

This makes it possible to acquire the distance information using the Time-of-Flight method. Since the Time-of-Flight method makes it possible to acquire the distance information from the sensor information output from the range sensor 214, or acquire the distance information merely by performing a simple correction process on the sensor information, the process is facilitated as compared with the case of using stereo matching or the like. Moreover, it is possible to suppress a situation in which the applied light enters the object (i.e., tissue in a narrow sense) by utilizing blue light having a short wavelength. Therefore, the distance information about the distance to the surface of the object is accurately calculated when extracting an concavity-convexity part present on the surface of the object as an concavity-convexity part of the object, and it is possible to improve the extraction accuracy, for example.

4. Third embodiment

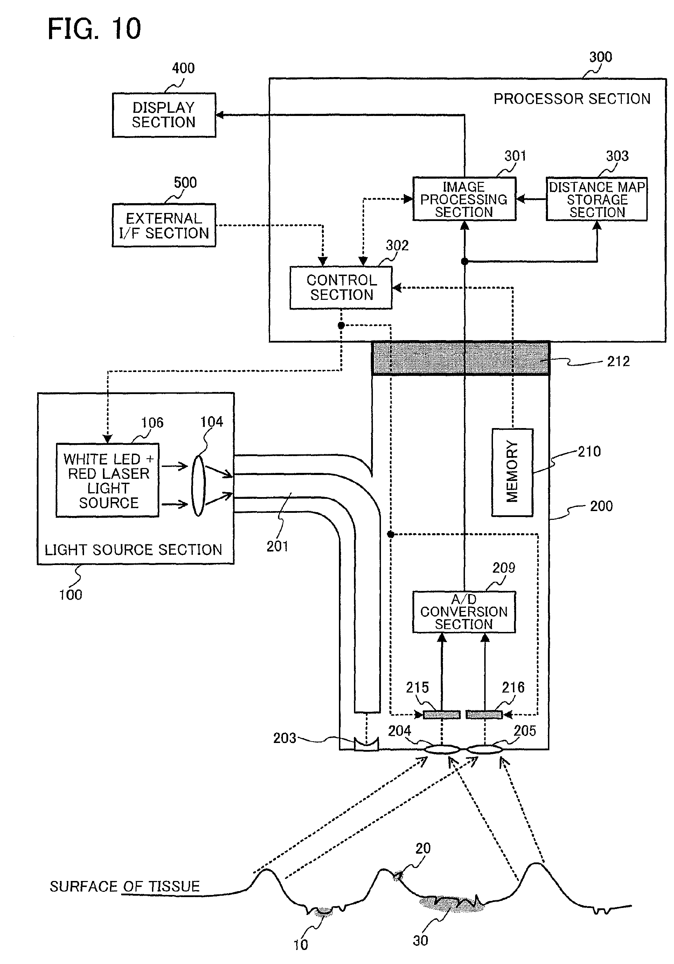

FIG. 10 is a functional block diagram illustrating an endoscope apparatus according to the third embodiment. The endoscope apparatus according to the third embodiment includes a light source section 100, an imaging section 200, a processor section 300, a display section 400, and an external I/F section 500.

The endoscope apparatus according to the third embodiment differs from the endoscope apparatus according to the second embodiment in that the light source section 100 includes a white LED and an infrared laser light source 106, and the imaging section 200 includes two image sensors 215 and 216. The image sensors 215 and 216 output a stereo image to the processor section 300 in the same manner as described above in connection with the first embodiment. At least one of the image sensors 215 and 216 is configured so that pixels of an infrared range sensor that utilizes the Time-of-Flight method are provided under RGB pixels of a primary-color single-chip image sensor. The stereo image is output to an image processing section 301, and a distance map detected using infrared light is output to a distance map storage section 303.

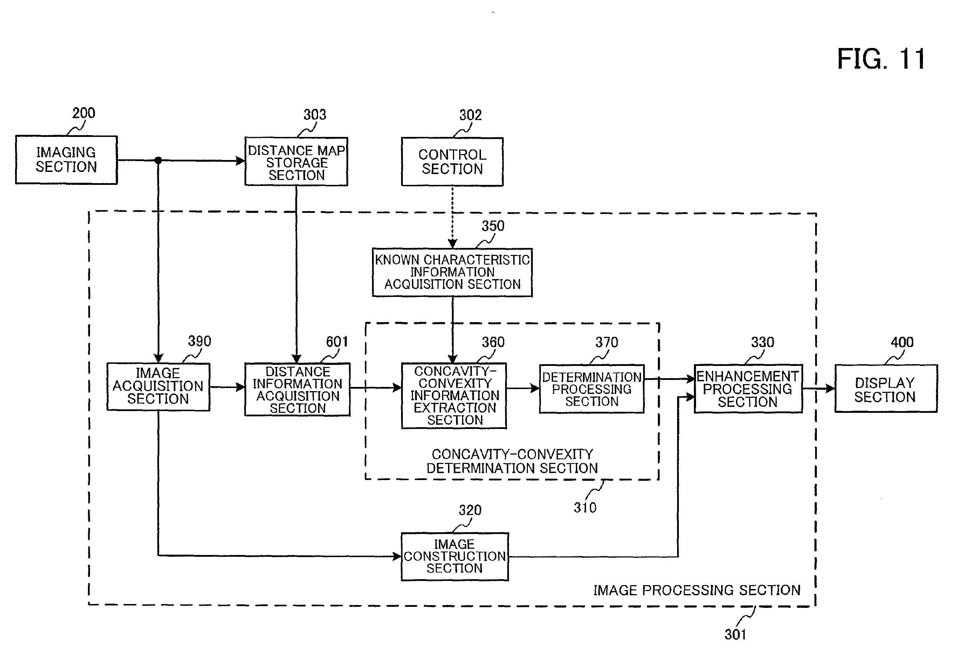

The details of the image processing section 301 are described below with reference to FIG. 11. The image processing section 301 includes an image acquisition section 390, an image construction section 320, a distance information acquisition section 601, a known characteristic information acquisition section 350, a concavity-convexity determination section 310, and an enhancement processing section 330, and the concavity-convexity determination section 310 includes a concavity-convexity information extraction section 360 and a determination processing section 370.

The stereo image (left image and right image) output from the image sensors 215 and 216 included in the imaging section 200 is acquired by the image acquisition section 390, and the acquired stereo image is input to the image construction section 320 and the distance information acquisition section 601.

The image construction section 320 performs given image processing (e.g., OB process, gain process, and $\gamma$ process) on the captured stereo image to generate an image that can be output to the display section 400. The resulting image is output to the enhancement processing section 330.

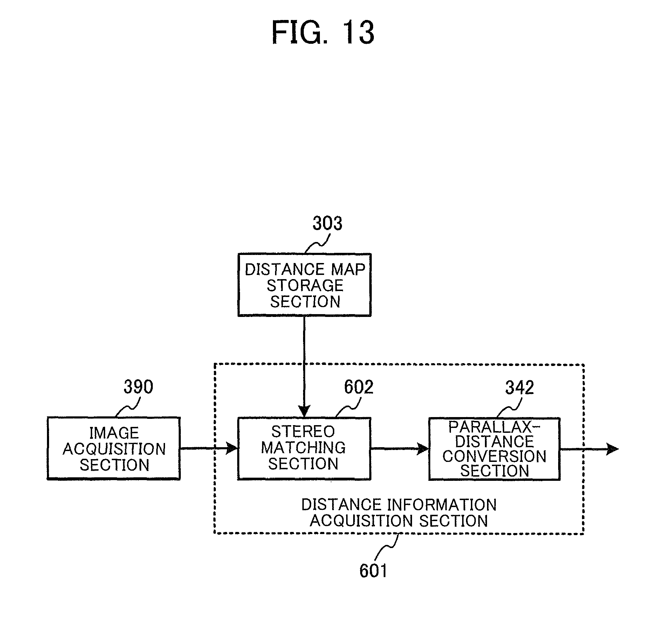

As illustrated in FIG. 13, the distance information acquisition section 601 includes a stereo matching section 602 and a parallax-distance conversion section 342. The stereo matching section 602 performs a matching calculation process on the left image (reference image) included in the captured stereo image and a local area of the right image along an epipolar line that passes through the attention pixel positioned at the center of a local area of the left image to calculate the position at which the maximum correlation is obtained as a parallax. In this case, the matching calculation process performs a search process on only the vicinity of the position of the parallax corresponding to the distance stored in the distance map storage section 303. Specifically, the search range when acquiring the distance information is limited using the distance map that is acquired using the Time-of-Flight method and stored in the distance map storage section 303, and the search process is performed on the limited search range when acquiring the distance information using the stereo image. Therefore, it is possible to perform the stereo matching process at a high speed, and prevent an erroneous determination. The parallax information is acquired by the matching process, and the distance information acquisition section 601 converts the acquired parallax information into the distance in the Z-direction, and outputs the resulting distance map to the concavity-convexity information extraction section 360.

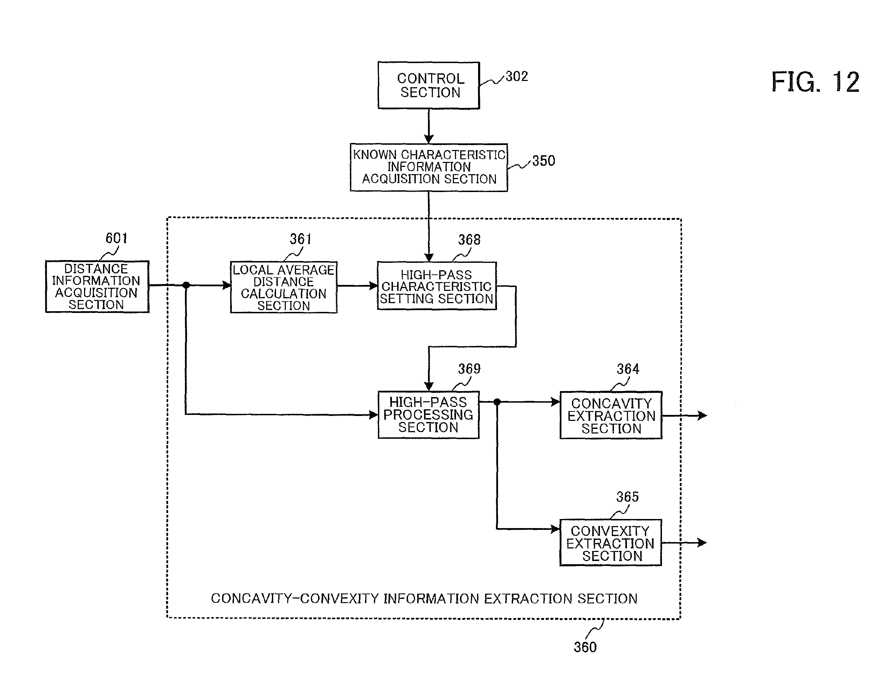

In the third embodiment, the concavity-convexity information extraction section 360 extracts the extracted concavity-convexity information using a high-pass filter. FIG. 12 illustrates the details of the concavity-convexity information extraction section 360. The distance map acquired by the distance information acquisition section 601 is input to a local average distance calculation section 361, and the local average distance calculation section 361 calculates the local average distance in the same manner as described above in connection with the first and second embodiments, and outputs the calculated local average distance to a high-pass characteristic setting section 368.

The size (i.e., dimensional information (e.g., width, height, or depth)) of the extraction target concavity-convexity part of tissue due to a lesion, the size (i.e., dimensional information (e.g., width, height, or depth)) of the lumen and the folds of the observation target part based on observation target part information, and the like are input to the high-pass characteristic setting section 368 from the known characteristic information acquisition section 350 as the known characteristic information in the same manner as described above in connection with the second embodiment. The optical magnification of the imaging section 200, and the local average distance are also input to the high-pass characteristic setting section 368. The size of the distance map (Z-direction) and the size of the object corresponding to the coordinate system (pixel pitch) orthogonal to the Z-direction are caused to coincide with each other, and the characteristics of the high-pass filter are determined so that the extraction target concavity-convexity part of tissue due to a lesion can be maintained, and the structure of the lumen and the folds of the observation target part can be cut off.

The filter characteristics of the high-pass filter are controlled using a cut-off frequency fhc, for example. The cut-off frequency fhc may be set so that the frequency F1 in the cycle D1 passes through, and the frequency F2 in the cycle D2 does not pass through. For example, the cut-off frequency fhc may be set to fhc=(F1+F2)/2*Rf. Note that Rf is a function that is designed so that the output value decreases as the local average distance decreases, and increases as the local average distance increases.

The high-pass filter characteristics are set on a pixel basis in the same manner as described above in connection with the second embodiment, and the high-pass processing section 369 can directly extract the extraction target concavity-convexity part due to a lesion. Specifically, the extracted concavity-convexity information is acquired directly (see FIG. 8C) without performing a subtraction process, and the acquired extracted concavity-convexity information is output to a concavity extraction section 364 and a convexity extraction section 365.

The concavity extraction section 364 extracts only an area having a negative sign from the extracted concavity-convexity information (concavity-convexity parts) to output a concavity image. The convexity extraction section 365 extracts only an area having a positive sign from the extracted concavity-convexity information (concavity-convexity parts) to output a convexity image.

The subsequent process is the same as described above in connection with the first and second embodiments, and description thereof is omitted.

According to the third embodiment, the stereo image and the range sensor that utilizes the Time-of-Flight method are provided, and the distance information about the surface of tissue is extracted using a red laser, and then accurately calculated using the stereo matching process. According to this configuration, since the matching range of the stereo matching process can be limited using the distance information, it is possible to reduce a matching determination error, and improve the processing speed.

According to the third embodiment, the distance information acquisition section 340 acquires low-accuracy provisional distance information that represents the distance from the imaging section 200 to the object, and acquires the distance information having high accuracy as compared with the provisional distance information based on the parallax information obtained from a plurality of captured images using the search range that is limited using the acquired provisional distance information.

This makes it possible to implement a reduction in processing load, a reduction in processing time, and the like when performing the process (stereo matching process) that acquires the parallax information, and calculates the distance information. When the search range is not limited, a huge amount of calculations are required, and the accuracy of the acquired distance information may decrease to a large extent when the matching process is performed in a state in which it is difficult to obtain matching results for some reason. If it is possible to acquire rough distance information although the accuracy thereof is lower than that of the final distance information that is used to extract the extracted concavity-convexity information, it is possible to suppress occurrence of the above problem by performing the search process utilizing the acquired information.

The imaging section 200 may include a light source section that emits infrared light (red laser light source 106), and a ranging device that receives reflected light that is the infrared light reflected by the object (see FIG. 10), and the distance information acquisition section 340 may acquire the provisional distance information based on time information about the time from the timing at which the infrared light was emitted from the light source section to the timing at which the ranging device received the reflected light.

In this case, the imaging section 200 may include an image sensor in which the ranging device is provided under a single-chip image sensor in which RGB pixels used to generate the captured image are provided. In FIG. 10, one of the image sensors 215 and 216 is such an image sensor.

This makes it possible to acquire the provisional distance information using the Time-of-Flight method that utilizes infrared light. Infrared light is widely used for the Time-of-Flight method. However, since infrared light has a long wavelength, infrared light may enter the object (tissue in a narrow sense) without being reflected by the surface of the object, and the infrared light that is scattered within the object may be detected by the ranging device. In this case, it may be difficult to acquire accurate distance information. However, it is possible to obtain sufficient information when infrared light is used to limit the search range of the stereo matching process. It is also possible to provide the ranging device under a normal image sensor (e.g., single-ship Bayer array image sensor). In this case, since it is unnecessary to separate image construction light and ranging light (see FIG. 10 in which the dichroic prism 217 illustrated in FIG. 6 is not provided), differing from the example illustrated in FIG. 6, it is possible to simplify the configuration of the imaging section 200, and reduce the size of the imaging section 200, for example.

5. Fourth embodiment

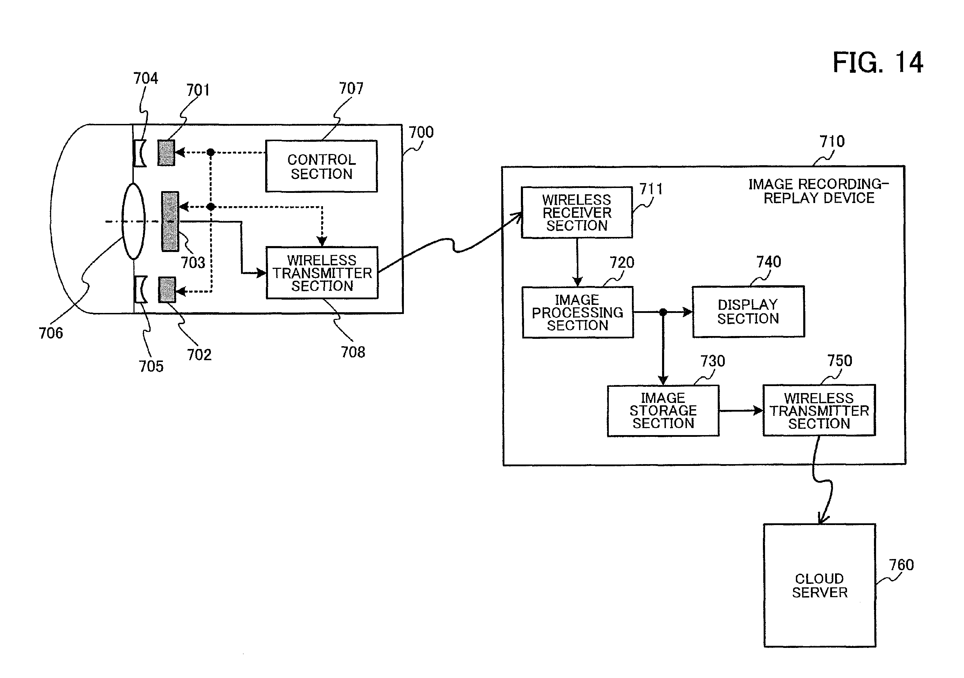

FIG. 14 is a functional block diagram illustrating a capsule endoscope apparatus according to the fourth embodiment. A capsule endoscope apparatus 700 according to the fourth embodiment includes a white LED 701, an infrared laser 702, an objective lens 706, illumination lenses 704 and 705, an image sensor 703 (i.e., an image sensor that includes a range sensor that utilizes the Time-of-Flight method (that uses infrared light)) similar to that used in connection with the third embodiment, a control section 707, and a wireless transmitter section 708.

The control section 707 controls the white LED 701 and the infrared laser 702 to emit light in a pulsed manner, and the image sensor 703 outputs the captured image and the distance map to the wireless transmitter section 708 in synchronization with the emission timing.

The wireless transmitter section 708 performs wireless communication with a wireless receiver section 711 included in an image recording-replay device 710 to transmit the captured image and the distance map to the image recording-replay device 710. The captured image and the distance map transmitted to the image recording-replay device 710 are output to an image processing section 720. The image processing section 720 performs a lesion recognition process based on the distance map and the captured image, and outputs the processing results to an image storage section 730 and a display section 740. The images stored in the image storage section 730 are transmitted to a server through the wireless transmitter section 750.

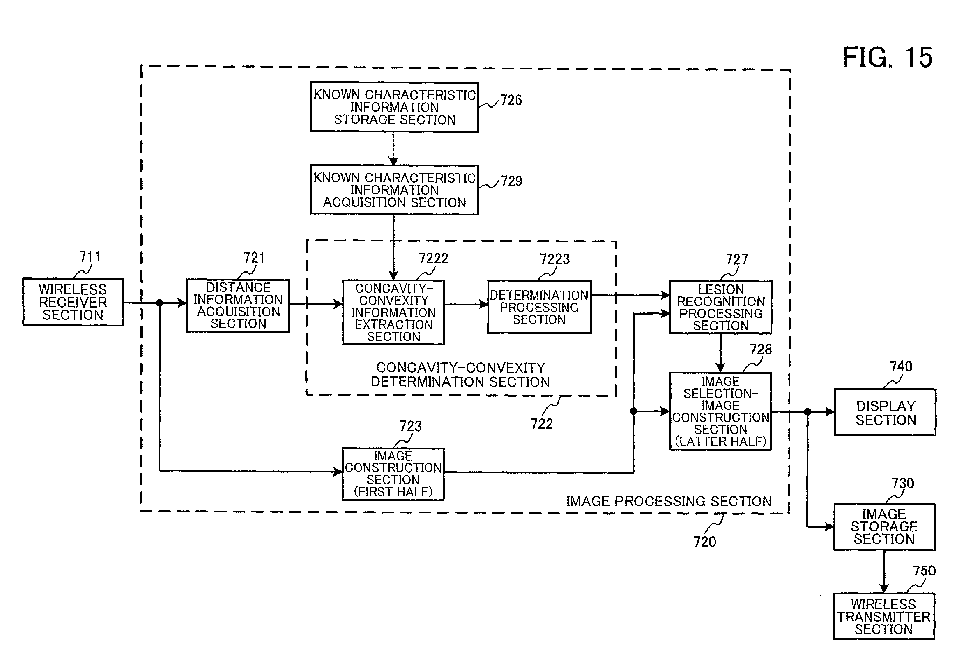

FIG. 15 illustrates the details of the image processing section 720. The image processing section 720 includes a distance information acquisition section 721, a known characteristic information acquisition section 729, a concavity-convexity determination section 722, an image construction section (first half) 723, a known characteristic information storage section 726, a lesion recognition processing section 727, and an image selection-image construction section (latter half) 728.

The concavity-convexity determination section 722 includes a concavity-convexity information extraction section 7222 and a determination processing section 7223 in the same manner as described above in connection with the first to third embodiments. Note that the process performed by the known characteristic information acquisition section 729 and the process performed by the determination processing section 7223 are respectively the same as the process performed by the known characteristic information acquisition section 350 and the process performed by the determination processing section 370 described above in connection with the first to third embodiments, and detailed description thereof is omitted. The process performed by the concavity-convexity information extraction section 7222 is the same as the process performed by the concavity-convexity information extraction section 360 described above in connection with the second embodiment, except that infrared light is used for the Time-of-Flight method, and description thereof is omitted.

The image construction section (first half) 723 performs an OB process, a WB process, a demosaicing process, and a color matrix process, and outputs the resulting image to the lesion recognition processing section 727 and the image selection-image construction section (latter half) 728.

The concavity-convexity determination section 722 outputs the concavity-convexity information to the lesion recognition processing section 727. The lesion recognition processing section 727 determines the presence or absence of a lesion based on the captured image from which an concavity-convexity part has been specified, and color information about the image corresponding to the concavity-convexity part.

The determination results are output to the image selection-image construction section (latter half) 728. The image selection-image construction section (latter half) 728 performs a $\gamma$ process, a scaling process, and an enhancement process on the image processed by the image construction section (first half) 723, and outputs the resulting image to the display section 740 and the image storage section 730.

According to the fourth embodiment, since the distance information is acquired using the range sensor that utilizes the Time-of-Flight method, an concavity-convexity part present on the surface of tissue can be used for the image recognition process, and the determination error rate of the image recognition process can be reduced. This makes it possible to delete unnecessary images, and effectively implement an image summarization process.

6. Fifth embodiment

FIG. 1 (see the first embodiment) is a functional block diagram illustrating an endoscope apparatus according to the fifth embodiment. As illustrated in FIG. 17A, a polyp 2 (i.e., elevated lesion) is present on the surface 1 of the large intestine (i.e., observation target), and a normal duct 40 and an abnormal duct 50 are present in the mucous membrane surface layer of the polyp 2. A recessed lesion 60 (in which the ductal structure has disappeared) is present at the base of the polyp 2. FIG. 17B is a schematic top view illustrating the polyp 2 present on the surface 1. The normal duct 40 has an approximately circular shape, and the abnormal duct 50 has a shape differing from that of the normal duct 40.

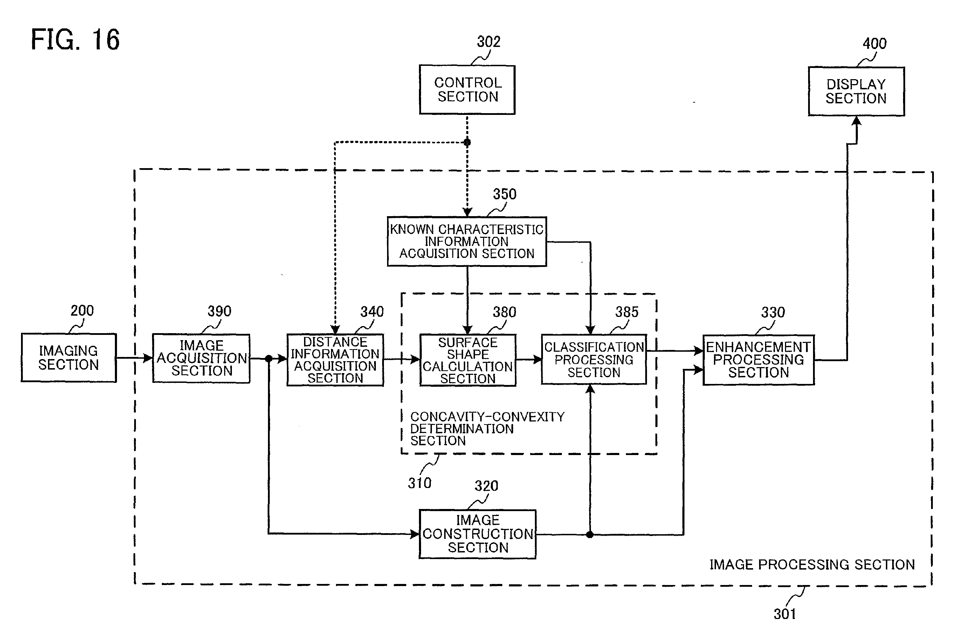

The details of the image processing section 301 are described below with reference to FIG. 16. The image processing section 301 includes an image acquisition section 390, an image construction section 320, a distance information acquisition section 340, a known characteristic information acquisition section 350, a concavity-convexity determination section 310, and an enhancement processing section 330 in the same manner as described above in connection with the first embodiment and the like. The configuration of the concavity-convexity determination section 310 differs from that illustrated in FIG. 3 (first embodiment). The concavity-convexity determination section 310 according to the fifth embodiment includes a surface shape calculation section 380 and a classification processing section 385. Note that description of the same configuration as that described above in connection with the first embodiment and the like is omitted. Each section included in the concavity-convexity determination section 310 is described below.

The surface shape calculation section 380 performs the closing process or the adaptive low-pass filtering process on the distance information (e.g., distance map) input from the distance information acquisition section 340 to extract a structure having a size equal to or larger than a given structural element. The given structural element is a classification target ductal structure formed on the surface 1 of the observation target part.

The known characteristic information acquisition section 350 acquires structural element information as the known characteristic information, and outputs the structural element information to the surface shape calculation section 380. Specifically, the structural element information is size information that is determined by the optical magnification of the imaging section 200 determined based on the scope ID information input from the memory 210, and the size (width information) of the ductal structure to be classified from the surface structure of the surface 1. The structural element information corresponds to the size of the ductal structure within the captured image when the ductal structure is captured at a given distance.

The observation target part is determined by the control section 302 based on the scope ID information input from the memory 210. For example, when the scope is an upper gastrointestinal scope, the observation target part is the gullet, the stomach, or the duodenum. When the scope is a lower gastrointestinal scope, the observation target part is the large intestine. A typical duct size is stored in the control section 302 in advance, and the information about the typical duct size is output to the surface shape calculation section 380 based on the observation target part. The observation target part may be determined using a method other than the method that utilizes the scope ID information. For example, the user may select the observation target part using a switch provided to the external I/F section 500.

The surface shape calculation section 380 adaptively generates surface shape calculation information based on the input distance information. The details of the surface shape calculation information are described later. For example, the surface shape calculation information may be the morphological kernel size (i.e., the size of the structural element) that is adapted to the distance information at the attention position on the distance map, or may be a low-pass filter that is adapted to the distance information. Specifically, the surface shape calculation information is change information that changes an adaptive nonlinear or linear low-pass filter corresponding to the distance information.

The generated surface shape information is input to the classification processing section 385 together with the distance map. The classification processing section 385 corrects a basic pit (binary image) obtained by modeling one normal ductal structure for classifying the ductal structure (pit pattern), and generates a corrected pit that is adapted to the three-dimensional shape of the surface of the tissue within the captured image as a classification reference. The terms "basic pit" and "corrected pit" are used since the pit pattern is the classification target. The terms "basic pit" and "corrected pit" can respectively be replaced by the term "reference pattern" and "corrected pattern" having a broader meaning.

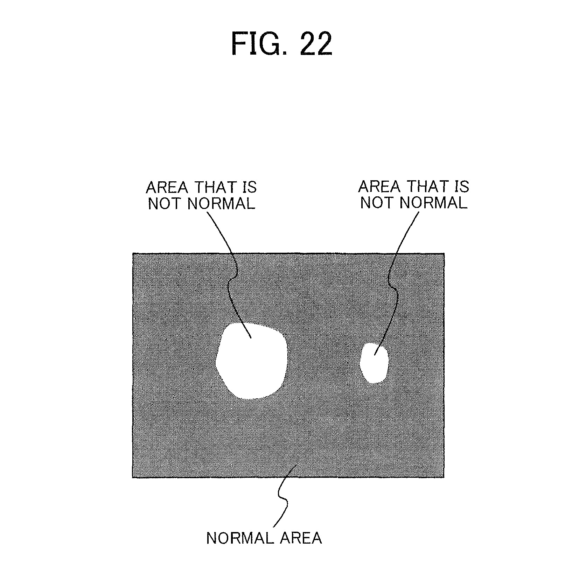

The classification processing section 385 performs a classification process using the generated classification reference (corrected pit). The image subjected to given image processing by the image construction section 320 is input to the classification processing section 385. The classification processing section 385 determines the presence or absence of the corrected pit within the captured image using a known pattern matching process, and outputs a classification map (binary image) (in which the classification areas are grouped) (see FIG. 22) to the enhancement processing section 330. The image (having the same size as that of the classification image) subjected to given image processing by the image construction section 320 is input to the enhancement processing section 330.

The enhancement processing section 330 performs the enhancement process on the image output from the image construction section 320 using the information that represents the classification results illustrated in FIG. 22.

The details of the surface shape calculation section 380 are described below with reference to FIGS. 18A and 18B. FIG. 18A illustrates the surface 1 of the object and the vertical cross section of the imaging section 200. FIG. 18A schematically illustrates a state in which the surface shape is calculated using the morphological process (closing process). The radius of the structural element (sphere) used for the closing process is set to be equal to or more than twice the size of the classification target ductal structure (surface shape calculation information). This aims at extracting a smoother three-dimensional surface shape of the surface 1 without extracting minute concavities-convexities of the normal duct 40, the abnormal duct 50, and the recessed lesion 60 (in which the ductal structure has disappeared), and reducing a correction error of the corrected pit obtained by correcting the basic pit.

FIG. 18B illustrates the cross section of the surface of the tissue after the closing process has been performed. FIG. 18B illustrates the results of a normal vector calculation process performed on the surface of the tissue. The normal vector is used as the surface shape information. Note that the surface shape information is not limited to the normal vector. The surface shape information may be the curved surface illustrated in FIG. 18B, or may be another piece of information that can represent the surface shape.

When implementing the above process, the size (e.g., width in the longitudinal direction) of the duct of tissue is acquired from the known characteristic information acquisition section 350 as the known characteristic information. It is possible to extract only the desired surface shape by determining the radius of the sphere applied to the surface of the tissue during the closing process using the above information. The radius of the sphere is set to be larger than the size of the duct.

The closing process is performed in the same manner as described above in connection with the first embodiment (except for the size of the structural element), and detailed description is omitted. The size of the structural element may be adaptively determined using the distance information, the imaging magnification of the imaging section 200, or the like in the same manner as described above in connection with the first embodiment.

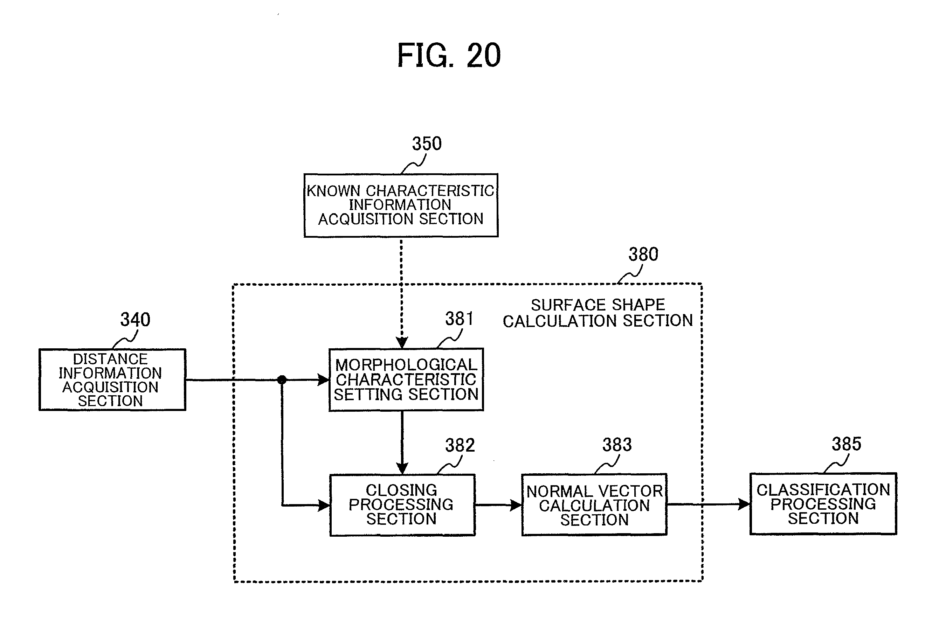

FIG. 20 is a detailed block diagram illustrating the surface shape calculation section 380. The surface shape calculation section 380 includes a morphological characteristic setting section 381, a closing processing section 382, and a normal vector calculation section 383.

The size (e.g., width in the longitudinal direction) of the duct of tissue (i.e., known characteristic information) is input to the morphological characteristic setting section 381 from the known characteristic information acquisition section 350, and the surface shape calculation information (e.g., the radius of the sphere used for the closing process) is determined.

Information about the radius of the sphere thus determined is input to the closing processing section 382 as a radius map having the same number of pixels as that of the distance map. The closing processing section 382 performs the closing process while changing the radius of the sphere on a pixel basis using the diameter map. The processing results of the closing processing section 382 are output to the normal vector calculation section 383.

The distance map after the closing process is input to the normal vector calculation section 383, and the normal vector calculation section 383 defines a plane using three-dimensional information about the attention sampling position and two sampling positions adjacent thereto on the distance map, and calculates the normal vector to the defined plane. The calculated normal vector is output to the classification processing section 385 as a normal vector map that is identical with the distance map as to the number of sampling points.

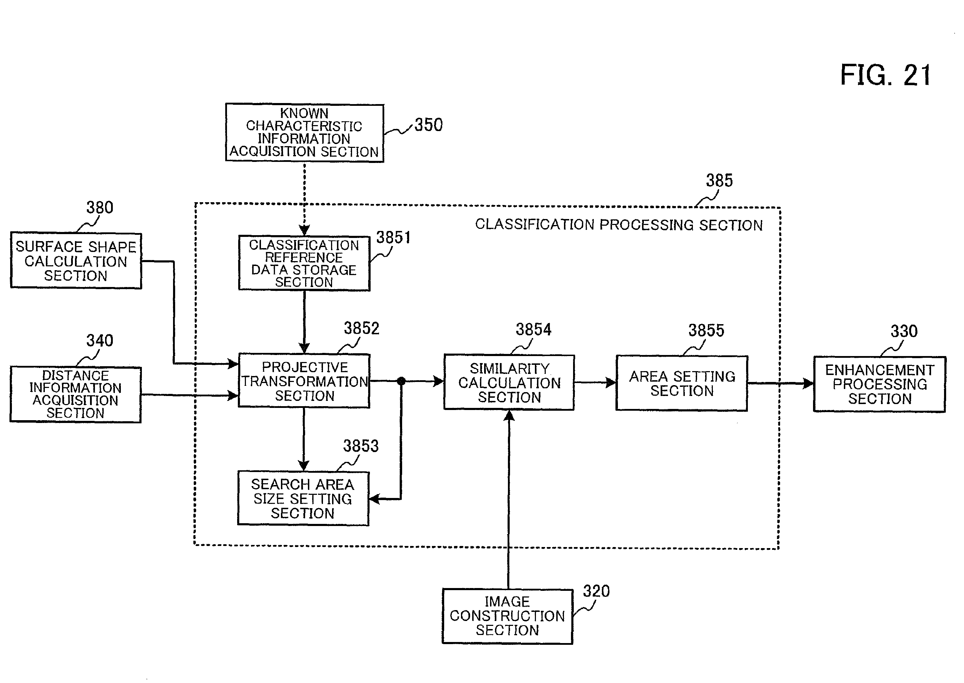

As illustrated in FIG. 21, the classification processing section 385 includes a classification reference data storage section 3851, a projective transformation section 3852, a search area size setting section 3853, a similarity calculation section 3854, and an area setting section 3855.

The classification reference data storage section 3851 stores the basic pit obtained by modeling the normal duct exposed on the surface of the tissue (see FIG. 19A). The basic pit is a binary image having a size corresponding to the size of the normal duct captured at a given distance. The basic pit is output to the projective transformation section 3852.

The distance map output from the distance information acquisition section 340, the normal vector map output from the surface shape calculation section 380, and the optical magnification output from the control section 302 are input to the projective transformation section 3852. The projective transformation section 3852 extracts the normal vector at the sampling position that corresponds to the distance information at the attention sampling position on the distance map, performs a projective transformation process on the basic pit, performs a magnification correction process corresponding to the optical magnification to generate a corrected pit, and outputs the corrected pit to the similarity calculation section 3854 as the classification reference. FIG. 19B illustrates an example of the corrected pit. The size of the corrected pit generated by the projective transformation section 3852 is output to the search area size setting section 3853.

The search area size setting section 3853 sets an area having a size twice the size of the corrected pit to be a search area of a similarity calculation process, and outputs the search area to the similarity calculation section 3854.

The corrected pit at the attention sampling position output from the projective transformation section 3852, and the search area corresponding to the corrected pit output from the search area size setting section 3853 are input to the similarity calculation section 3854, and the similarity calculation section 3854 extracts the search area from the image (subjected to given image processing) output from the image construction section 320.

The similarity calculation section 3854 performs a high-pass filtering process or a band-pass filtering process on the image of the extracted search area to remove a low-frequency component, and performs a binarization process to generate a binary search area. The similarity calculation section 3854 performs a pattern matching process (that calculates the correlation value by calculating the sum of absolute differences) within the binary search area using the corrected pit (classification reference) to calculate the correlation value, and outputs the peak position and a maximum correlation value (minimum value of the sum of absolute differences) map to the area setting section 3855. Note that the correlation value may be calculated using a phase-only correlation (POC) method or the like. Since rotation and a change in magnification become invariable when using the POC method, it is possible to improve the correlation calculation accuracy.

The area setting section 3855 calculates an area in which the sum of absolute differences is equal to or less than a threshold value T based on the maximum correlation value map input from the similarity calculation section 3854, and calculates the three-dimensional distance between the position within the calculated area that corresponds to the maximum correlation value and the position within the adjacent search range that corresponds to the maximum correlation value. When the calculated three-dimensional distance is included within a given error range, the area setting section 3855 performs a grouping process using the area including the maximum correlation position as a normal area, and outputs the classification map illustrated in FIG. 22 to the enhancement processing section 330.

According to the fifth embodiment, since the classification process corresponding to a change in pit shape of the normal duct based on the surface shape of tissue is performed, it is possible to improve the accuracy of classification from the abnormal duct area.

According to the fifth embodiment, the concavity-convexity determination section 310 includes the surface shape calculation section 380 that calculates the surface shape information about the object based on the distance information and the known characteristic information, and the classification processing section 385 that generates the classification reference based on the surface shape information, and performs the classification process that utilizes the generated classification reference (see FIG. 16). The concavity-convexity determination section 310 performs the classification process that utilizes the classification reference as the concavity-convexity determination process.

This makes it possible to calculate the surface shape information based on the distance information and the known characteristic information, and specify an concavity-convexity part by performing the classification process that utilizes the classification reference generated using the surface shape information. This makes it possible to adaptively generate the classification reference based on the surface shape represented by surface shape information, and perform the classification process, for example. A decrease in the accuracy of the classification process due to the surface shape may occur due to deformation of the structure within the captured image caused by the angle formed by the optical axis direction of the imaging section 200 and the surface of the object, for example. The method according to the fifth embodiment makes it possible to accurately perform the classification process (concavity-convexity determination process) even in such a situation.

The known characteristic information acquisition section 350 may acquire the reference pattern that corresponds to the structure of the object in a given state as the known characteristic information, and the classification processing section 385 may generate the corrected pattern as the classification reference, and perform the classification process using the generated classification reference, the corrected pattern being acquired by performing a deformation process based on the surface shape information on the reference pattern.

According to this configuration, since the classification process can be performed using the corrected pattern (obtained by performing the deformation process based on the surface shape information on the reference pattern acquired as the known characteristic information) as the classification reference, it is possible to accurately perform the classification process even when the structure of the object is captured in a deformed state due to the surface shape. Specifically, a circular ductal structure may be captured in a variously deformed state (see FIG. 17B). It is possible to appropriately detect and classify the pit pattern even in a deformed area by generating an appropriate corrected pattern (corrected pit in FIG. 19B) from the reference pattern (basic pit in FIG. 19A) corresponding to the surface shape, and using the generated corrected pattern as the classification reference. Note that the deformation process based on the surface shape information is performed by the projective transformation section 3852 illustrated in FIG. 21, for example.

The classification processing section 385 may calculate the similarity between the structure of the object captured within the captured image and the corrected pattern used as the classification reference at each position within the captured image, and perform the classification process based on the calculated similarity.

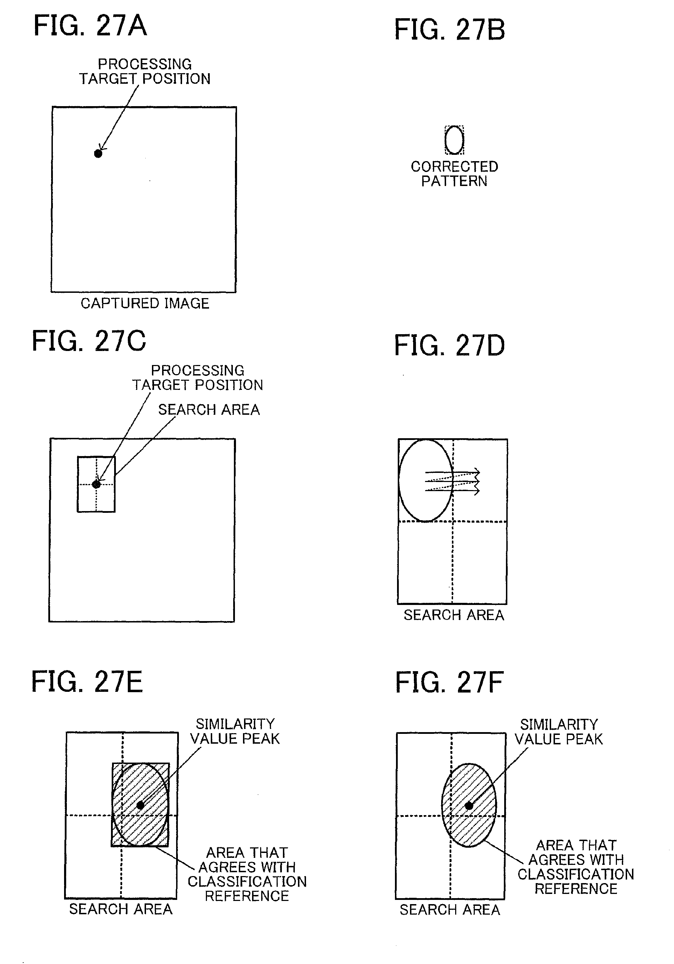

This makes it possible to perform the classification process using the similarity between the structure within the captured image and the classification reference (corrected pattern). FIGS. 27A to 27F illustrate a specific example. When one position within the image is set to the processing target position (see FIG. 27A), a corrected pattern at the processing target position is acquired by deforming the reference pattern based on the surface shape information at the processing target position (see FIG. 27B). A search area (e.g., an area having a size twice the size of the corrected pattern) is set around the processing target position based on the corrected pattern (see FIG. 27C), and the matching process is performed on the captured structure and the corrected pattern within the search area (see FIG. 27D). When the matching process is performed on a pixel basis, the similarity is calculated on a pixel basis. A pixel that corresponds to the peak of the similarity within the search area is specified (see FIG. 27E), and whether or not the similarity at the specified pixel is equal to or larger than a given threshold value is determined. When the similarity at the specified pixel is equal to or larger than the threshold value (i.e., when the corrected pattern has been detected within the area having the size of the corrected pattern based on the peak position (the center of the corrected pattern is set to be the reference position in FIG. 27E)), it is determined that the area agrees with the reference pattern. Note that the inside of the shape that represents the corrected pattern may be determined to be the area that agrees with the classification reference (see FIG. 27F). Various modifications may be made. When the similarity at the specified pixel is less than the threshold value, it is determined that a structure that matches the reference pattern is not present in the area around the processing target position. An area (0, 1, or a plurality of areas) that agrees with the reference pattern, and an area other than the area that agrees with the reference pattern are set within the captured image by performing the above process at each position within the image. When a plurality of areas agree with the reference pattern, overlapping areas and contiguous areas among the plurality of areas are integrated to obtain the classification results illustrated in FIG. 22. Note that the classification process based on the similarity described above is only an example. The classification process may be performed using another method. The similarity may be calculated using various known methods that calculate the similarity between images or the difference between images, and detailed description thereof is omitted.

The known characteristic information acquisition section 350 may acquire the reference pattern that corresponds to the structure of the object in a normal state as the known characteristic information.

This makes it possible to implement the classification process that classifies the captured image into a normal area and an abnormal area (see FIG. 22). The term "abnormal area" refers to an area that is considered to be a lesion when using a medical endoscope, for example. Since it is considered that the user pays attention to such an area, it is possible to suppress a situation in which the attention area is missed, by appropriately classifying the captured image.

The object may include a global three-dimensional structure, and a local concavity-convexity structure that is more local than the global three-dimensional structure, and the surface shape calculation section 380 may calculate the surface shape information by extracting the global three-dimensional structure included in the object from the distance information without extracting the local concavity-convexity structure included in the object.

This makes it possible to calculate the surface shape information from the global structure when the structures of the object are classified into a global structure and a local structure. In the fifth embodiment, the classification reference is generated based on the surface shape information. Even if an concavity-convexity structure smaller than the reference pattern is present, the effects of such an concavity-convexity structure (e.g., deformation of the reference pattern within the captured image) are small, and a decrease in accuracy of the classification process predominantly occurs due to a global structure that is larger than the reference pattern. A decrease in accuracy of the reference pattern deformation process (corrected pattern calculation process) may occur if the classification reference is generated using a local concavity-convexity structure. For example, when the surface of the object is vertical to the optical axis direction of the imaging section 200, and a concavity smaller than the reference pattern is formed in the surface of the object, it is considered that the structure of the object that corresponds to the reference pattern is captured within the resulting image to have a shape identical with (or sufficiently close to) the shape of the reference pattern, and the matching process can be performed using the reference pattern. However, when the information about the local concavity is also used, the angle formed by the surface of the object and the optical axis direction in the area of the concavity significantly differs from 90°, and the corrected pattern (classification reference) is unnecessarily deformed in an area around the concavity. Therefore, the fifth embodiment implements an accurate classification process by calculating the surface shape information from a global three-dimensional structure.

The surface shape calculation section 380 may calculate the normal vector to the surface of the object represented by the global three-dimensional structure as the surface shape information.

This makes it possible to use the normal vector to the surface of the object (i.e., the normal vector orthogonal to the surface of the object in a narrow sense) as the surface shape information (see FIG. 18B). Note that the surface shape information is not limited to the normal vector. The surface shape information may be the surface that represents the global three-dimensional structure (e.g., information that represents the results of the closing process in FIG. 18B), or may be a set of tangents to the surface of the object, or may be another piece of information that can represent the surface shape.

The known characteristic information acquisition section 350 may acquire the reference pattern that corresponds to the structure of the object in a given state as the known characteristic information, and the classification processing section 385 may generate the corrected pattern as the classification reference, and perform the classification process using the generated classification reference, the corrected pattern being acquired by performing a deformation process based on the angle of the normal vector with respect to a given reference direction on the reference pattern.

This makes it possible to generate the classification reference by performing the deformation process that utilizes the direction of the normal vector when the normal vector is calculated as the surface shape information. The given reference direction refers to the optical axis direction of the imaging section 200, or a direction that is determined by the optical axis direction of the imaging section 200. It is possible to estimate the degree of deformation of the structure of the object at a position corresponding to the normal vector (when the structure is captured within the captured image) by utilizing the angle formed by the reference direction and the normal vector. Therefore, it is possible to accurately perform the classification process by performing the deformation process on the reference pattern using the estimation results (see FIGS. 19A and 19B).

7. Sixth embodiment

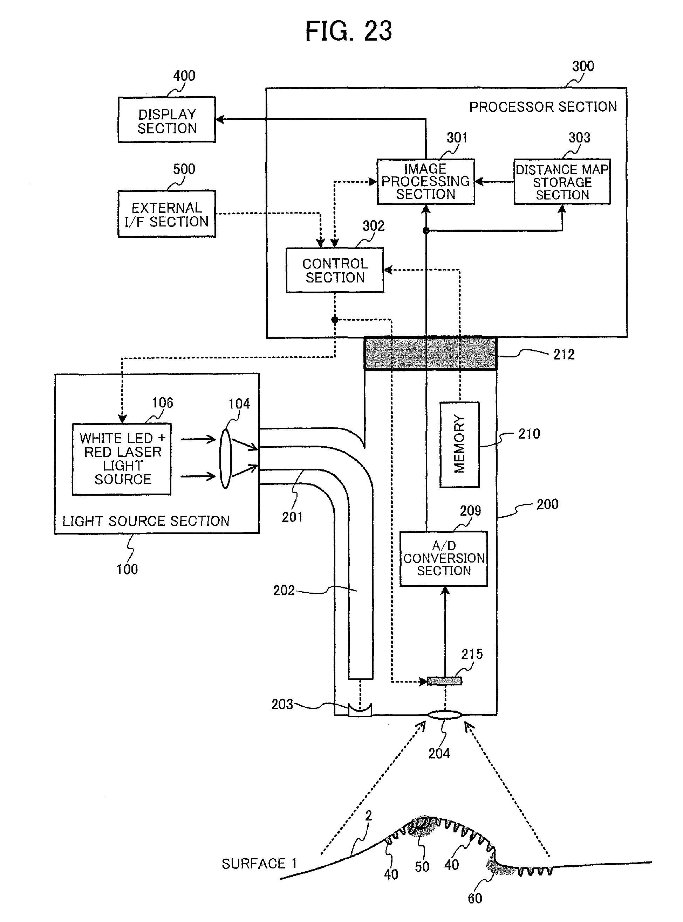

FIG. 23 is a functional block diagram illustrating an endoscope apparatus according to the sixth embodiment. The endoscope apparatus according to the sixth embodiment includes a light source section 100, an imaging section 200, a processor section 300, a display section 400, and an external I/F section 500.

The endoscope apparatus according to the sixth embodiment differs from the endoscope apparatus according to the third embodiment in that the light source section 100 includes a white LED and an infrared laser light source 106, and the imaging section 200 includes one image sensor 215 that is configured so that pixels of an infrared range sensor that utilizes the Time-of-Flight method are provided under RGB pixels of a primary-color single-chip image sensor. An image captured by the image sensor 215 is output to an image processing section 301, and a distance map detected using infrared light is output to a distance map storage section 303.

The configuration of the image processing section 301 according to the sixth embodiment is the same as the configuration of the image processing section 301 according to the fifth embodiment (see FIG. 16), and detailed description thereof is omitted. The process performed by the surface shape calculation section 380 is the same as that described above in connection with the fifth embodiment. Note that various modifications may be made, such as using a filtering process (see the second embodiment) instead of the morphological process.

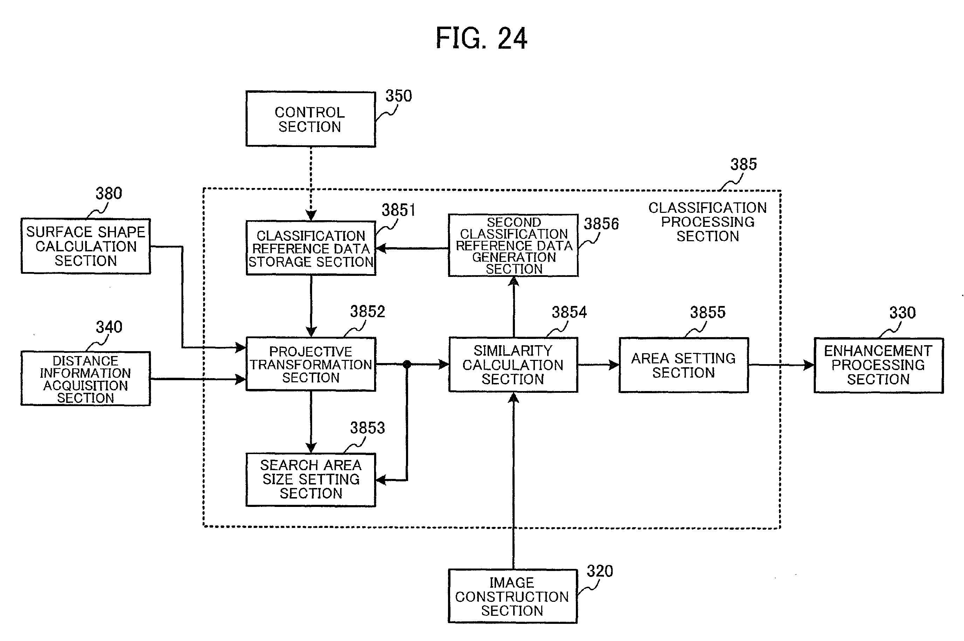

FIG. 24 illustrates a configuration example of the classification processing section 385 according to the sixth embodiment. The classification processing section 385 according to the sixth embodiment differs from the classification processing section 385 according to the fifth embodiment (see FIG. 21) in that a second classification reference data generation section 3856 is additionally provided.

The sixth embodiment differs from the fifth embodiment in that the basic pit (classification reference) is provided corresponding to the normal duct and the abnormal duct, a pit is extracted from the actual captured image, and used as second classification reference data (second reference pattern), and the similarity is calculated based on the second classification reference data.

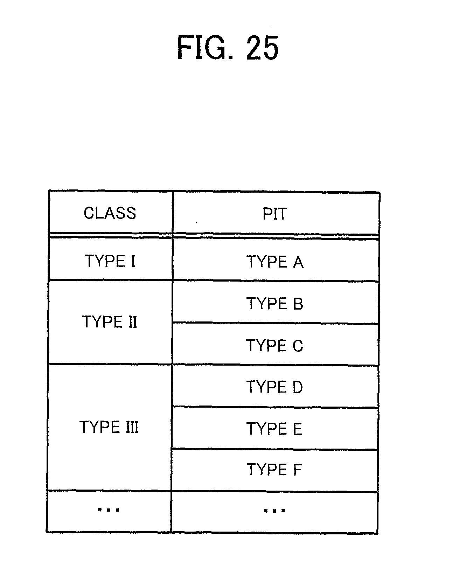

The differences between the sixth embodiment and the fifth embodiment are described in detail below. A plurality of pits including a basic pit corresponding to the normal duct (see FIG. 25) are stored in the classification reference data storage section 3851 included in the classification processing section 385, and output to the projective transformation section 3852. The process performed by the projective transformation section 3852 is the same as described in connection with the fifth embodiment. The projective transformation section 3852 performs the projective transformation process on each pit stored in the classification reference data storage section 3851, and output the corrected pits corresponding to a plurality of classification types to the search area size setting section 3853 and the similarity calculation section 3854.

The similarity calculation section 3854 generates the maximum correlation value map corresponding to each corrected pit. In the sixth embodiment, the maximum correlation value map is not used to generate the classification map (i.e., the final output of the classification process), but is output to the second classification reference data generation section 3856, and used to generate additional classification reference data.

The second classification reference data generation section 3856 sets the pit image at a position within the image for which the similarity calculation section 3854 has determined that the similarity is high (i.e., the absolute difference is equal to or smaller than a given value) to be the classification reference. This makes it possible to implement a more optimum and accurate classification (determination) process as compared with the case of using a typical pit model provided in advance.

More specifically, the maximum correlation value map (corresponding to each type) output from the similarity calculation section 3854, the distance map output from the distance information acquisition section 340, the optical magnification output from the control section 302, and the duct size (corresponding to each type) output from the known characteristic information acquisition section 350 are input to the second classification reference data generation section 3856. The second classification reference data generation section 3856 extracts the image data corresponding to the maximum correlation value sampling position (corresponding to each type) based on the distance information at the maximum correlation value sampling position, the size of the duct, and the optical magnification.

The second classification reference data generation section 3856 acquires a grayscale image (that cancels the difference in brightness) obtained by removing a low-frequency component from the extracted (actual) image, and outputs the grayscale image to the classification reference data storage section 3851 as the second classification reference data together with the normal vector and the distance information, and the classification reference data storage section 3851 stores the second classification reference data and relevant information. The second classification reference data having a high correlation with the object has thus been collected corresponding to each type.

Note that the second classification reference data includes the effects of the angle formed by the optical axis direction of the imaging section 200 and the surface of the object, and the effects of deformation depending on the distance from the imaging section 200 to the surface of the object. Therefore, the second classification reference data generation section 3856 may generate the second classification reference data after performing a process that cancels these effects. Specifically, the results of a deformation process (projective transformation process and scaling process) performed on the grayscale image so as to achieve a state in which the image is captured at a given distance from a given reference direction may be used as the second classification reference data.

After the second classification reference data has been generated, the projective transformation section 3852, the search area size setting section 3853, and the similarity calculation section 3854 perform the process on the second classification reference data. Specifically, the projective transformation process is performed on the second classification reference data to generate a second corrected pattern, and the process described above in connection with the fifth embodiment is performed using the generated second corrected pattern as the classification reference.

Note that the basic pit corresponding to the abnormal duct used in connection with the sixth embodiment is not normally point-symmetrical. Therefore, it is desirable that the similarity calculation section 3854 calculate the similarity (when using the corrected pattern or the second corrected pattern) by performing the rotation-invariant phase-only correction (POC).

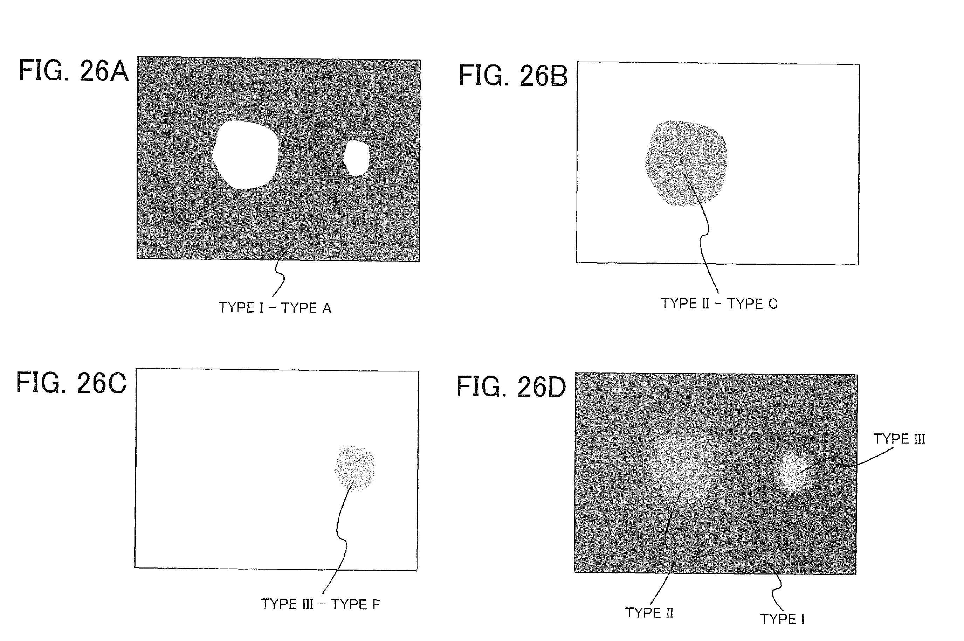

The area setting section 3855 generates the classification maps illustrated in FIGS. 26A to 26D. FIG. 26A illustrates an area in which a correlation is obtained by the corrected pit classified as the normal duct, and FIGS. 26B and 26C illustrate an area in which a correlation is obtained by the corrected pit classified as a different abnormal duct. FIG. 26D illustrates a classification map obtained by synthesizing three classification maps (multivalued image). The overlapping area of the areas in which a correlation is obtained corresponding to each type may be set to an unclassified area, or may be set to the type with a higher malignant level. The synthesized classification map illustrated in FIG. 26D is output to the enhancement processing section 330 from the area setting section 3855.

The enhancement processing section 330 performs the enhancement process (e.g., luminance enhancement process or color enhancement process) based on the classification map (multivalued image).

The remaining processes are performed in the same manner as described in connection with the fifth embodiment. According to the sixth embodiment, since the classification process is performed using the patterns corresponding to the normal duct and various abnormal ducts, and the classification reference is acquired from the captured image instead of using an average classification reference, it is possible to improve the accuracy of the classification process.

According to the sixth embodiment, the known characteristic information acquisition section 350 acquires the reference pattern that corresponds to the structure of the object in an abnormal state as the known characteristic information.

This makes it possible to acquire a plurality of reference patterns (see FIG. 25), generate the classification reference using the plurality of reference patterns, and perform the classification process, for example. The classification process may be performed in various ways. For example, first to Nth (N is an integer equal to or larger than 2) classification references may be generated from first to Nth reference patterns, the captured image may be classified into an area that agrees with the classification reference and an area that does not agree with the classification reference corresponding to each classification reference, and the results may be integrated. FIGS. 26A to 26C illustrate an example of the processing result obtained using each classification reference, and FIG. 26D illustrates an example of the integration results (i.e., the output of the classification process).

The known characteristic information acquisition section 350 may acquire the reference pattern that corresponds to the structure of the object in a given state as the known characteristic information, and the classification processing section 385 may perform the deformation process based on the surface shape information on the reference pattern to acquire the corrected pattern, calculate the similarity between the structure of the object captured within the captured image and the corrected pattern at each position within the captured image, and acquire a second reference pattern candidate based on the calculated similarity. The classification processing section 385 may generate the second reference pattern as a new reference pattern based on the acquired second reference pattern candidate and the surface shape information, perform the deformation process based on the surface shape information on the second reference pattern to generate the second corrected pattern as the classification reference, and perform the classification process using the generated classification reference.

This makes it possible to generate the second reference pattern based on the captured image, and perform the classification process using the second reference pattern. Since the classification reference can be generated from the object captured within the captured image, the classification reference sufficiently reflects the characteristics of the processing target object, and it is possible to improve the accuracy of the classification process as compared with the case of directly using the reference pattern acquired as the known characteristic information.

The first to sixth embodiments to which the invention is applied, and the modifications thereof have been described above. Note that the invention is not limited to the first to sixth embodiments and the modifications thereof. Various modifications and variations may be made without departing from the scope of the invention. A plurality of elements described above in connection with the first to sixth embodiments and the modifications thereof may be appropriately combined to achieve various configurations. For example, an arbitrary element may be omitted from the elements described above in connection with the first to sixth embodiments and the modifications thereof. The elements described above in connection with the first to sixth embodiments and the modifications thereof may be appropriately combined. Specifically, various modifications and applications are possible without materially departing from the novel teachings and advantages of the invention.

REFERENCE SIGNS LIST

10, 20, 30: lesion, 40: normal duct, 50: abnormal duct, 60: duct disappearance area, 100: light source section, 101: white light source, 102: rotary color filter, 103: rotation driver section, 104: condenser lens, 105: blue laser light source, 106: infrared laser light source, 200: imaging section, 201: light guide fiber, 203: illumination lens, 204: objective lens, 206, 215, 216: image sensor, 209: A/D conversion section, 210: memory, 212: connector, 213: image sensor, 214: range sensor, 217: dichroic prism, 300: processor section, 301: image processing section, 302: control section, 303: distance map storage section, 310: concavity-convexity determination section, 320: image construction section, 330: enhancement processing section, 340: distance information acquisition section, 341: stereo matching section, 342: parallax-distance conversion section, 350: known characteristic information acquisition section, 360: concavity-convexity information extraction section, 361: local average distance calculation section, 362: morphological characteristic setting section, 363-1: closing processing section, 363-2: opening processing section, 364: concavity extraction section, 365: convexity extraction section, 366: low-pass characteristic setting section, 367: low-pass processing section, 368: high-pass characteristic setting section, 369: high-pass processing section, 370: determination processing section, 380: surface shape calculation section, 381: morphological characteristic setting section, 382: closing processing section, 383: normal vector calculation section, 385: classification processing section, 390: image acquisition section, 3851: classification reference data storage section, 3852: projective transformation section, 3853: search area size setting section, 3854: similarity calculation section, 3855: area setting section, 3856: classification reference data generation section, 400: display section, 500: external I/F section, 601: distance information acquisition section, 700: capsule endoscope apparatus, 701: white LED, 702: infrared laser, 703: image sensor, 704: illumination lens, 706: objective lens, 707: control section, 708: wireless transmitter section, 710: replay device, 711: wireless receiver section, 720: image processing section, 721: distance information acquisition section, 722: concavity-convexity determination section, 726: known characteristic information storage section, 727: lesion recognition processing section, 729: known characteristic information acquisition section, 730: image storage section, 740: display section, 750: wireless transmitter section, 7222: concavity-convexity information extraction section, 7223: determination processing section

**Claims**

1. An image processing device comprising:

   an image acquisition section that acquires a captured image that includes an image of an object, the captured image being an image captured by an imaging section;
   a distance information acquisition section that acquires distance information based on a distance from the imaging section to the object when the imaging section captured the captured image;
   a known characteristic information acquisition section that acquires known characteristic information, the known characteristic information being information that represents known characteristics relating to a structure of the object; and
   a concavity-convexity determination section that performs a concavity-convexity determination process that specifies an concavity-convexity part of the object that agrees with characteristics specified by the known characteristic information, from the object captured within the captured image, based on the distance information and the known characteristic information.

2. The image processing device as defined in claim 1,
   the concavity-convexity determination section including a concavity-convexity information extraction section that extracts extracted concavity-convexity information that represents the concavity-convexity part of the object that agrees with the characteristics specified by the known characteristic information from the distance information, based on the distance information and the known characteristic information, and
   the concavity-convexity determination section performing the concavity-convexity determination process based on the extracted concavity-convexity information.

3. The image processing device as defined in claim 2,
the concavity-convexity information extraction section determining an extraction process parameter based on the known characteristic information, and extracting the concavity-convexity part of the object as the extracted concavity-convexity information based on the determined extraction process parameter.

4. The image processing device as defined in claim 3,
the known characteristic information acquisition section acquiring type information and concavity-convexity characteristic information as the known characteristic information, the type information being information that represents a type of the object, and the concavity-convexity characteristic information being information about the concavity-convexity part of the object that is linked to the type information, and
the concavity-convexity information extraction section determining the extraction process parameter based on the type information and the concavity-convexity characteristic information, and extracting the concavity-convexity part of the object as the extracted concavity-convexity information based on the determined extraction process parameter.

5. The image processing device as defined in claim 3,
the captured image being an *in vivo* image that is obtained by capturing the inside of a living body,
the known characteristic information acquisition section acquiring part information and concavity-convexity characteristic information as the known characteristic information, the part information being information that represents a part of the living body to which the object corresponds, and the concavity-convexity characteristic information being information about the concavity-convexity part of the living body, and
the concavity-convexity information extraction section determining the extraction process parameter based on the part information and the concavity-convexity characteristic information, and extracting the concavity-convexity part of the object as the extracted concavity-convexity information based on the determined extraction process parameter.

6. The image processing device as defined in claim 3,
the concavity-convexity information extraction section determining a size of a structural element used for an opening process and a closing process as the extraction process parameter based on the known characteristic information, and performing the opening process and the closing process using the structural element having the determined size to extract the concavity-convexity part of the object as the extracted concavity-convexity information.

7. The image processing device as defined in claim 6,
the concavity-convexity information extraction section decreasing the size of the structural element used as the extraction process parameter as a value represented by the distance information that corresponds to a processing target pixel of the opening process and the closing process increases.

8. The image processing device as defined in claim 3,
the concavity-convexity information extraction section determining frequency characteristics of a filter used for a filtering process performed on the distance information as the extraction process parameter based on the known characteristic information, and performing the filtering process that utilizes the filter having the determined frequency characteristics to extract the concavity-convexity part of the object as the extracted concavity-convexity information.

9. The image processing device as defined in claim 2,
the object including a global three-dimensional structure, and a local concavity-convexity structure that is more local than the global three-dimensional structure, and
the concavity-convexity information extraction section extracting the concavity-convexity part of the object that is selected from the global three-dimensional structure and the local concavity-convexity structure included in the object, and agrees with the characteristics specified by the known characteristic information, as the extracted concavity-convexity information.

10. The image processing device as defined in claim 2,
the captured image being an *in vivo* image that is obtained by capturing the inside of a living body,
the object including a global three-dimensional structure that is a lumen structure inside the living body, and a local concavity-convexity structure that is formed on the lumen structure, and is more local than the global three-dimensional structure, and
the concavity-convexity information extraction section extracting the concavity-convexity part of the object that is selected from the global three-dimensional structure and the local concavity-convexity structure included in the object, and agrees with the characteristics specified by the known characteristic information, as the extracted concavity-convexity information.

11. The image processing device as defined in claim 1,
the distance information acquisition section acquiring a distance map as the distance information, the distance map being a map in which information about the distance from the imaging section to the object captured at each pixel of the acquired captured image is linked to each pixel of the acquired captured image.

12. The image processing device as defined in claim 1,
the imaging section including a plurality of viewpoints,
the image acquisition section acquiring a plurality of the captured images that respectively correspond to the plurality of viewpoints, and
the distance information acquisition section acquiring the distance information based on parallax information obtained from the plurality of captured images acquired by the image acquisition section.

13. The image processing device as defined in claim 12,
the distance information acquisition section acquiring low-accuracy provisional distance information that represents the distance from the imaging section to the object, and acquiring the distance information having high accuracy as compared with the provisional distance information based on the parallax information obtained from the plurality of captured images using a search range that is limited using the acquired provisional distance information.

14. The image processing device as defined in claim 13,
the imaging section including a light source section that emits infrared light, and a ranging device that receives reflected light that is the infrared light reflected by the object, and
the distance information acquisition section acquiring the provisional distance information based on time information about a time from a timing at which the infrared light was emitted from the light source section to a timing at which the ranging device received the reflected light.

15. The image processing device as defined in claim 14,
the imaging section including an image sensor in which the ranging device is provided under a single-chip image sensor in which RGB pixels used to generate the captured image are provided.

16. The image processing device as defined in claim 1,
the imaging section including a light source section that emits blue light, and a ranging device that receives reflected light that is the blue light reflected by the object, and
the distance information acquisition section acquiring the distance information based on time information about a time from a timing at which the blue light was emitted from the light source section to a timing at which the ranging device received the reflected light.

17. The image processing device as defined in claim 1,
the concavity-convexity determination section including:

a surface shape calculation section that calculates surface shape information about the object based on the distance information and the known characteristic information; and
a classification processing section that generates a classification reference based on the surface shape information, and performs a classification process that utilizes the generated classification reference, and
the concavity-convexity determination section performing the classification process that utilizes the classification reference as the concavity-convexity determination process.

18. The image processing device as defined in claim 17,
the known characteristic information acquisition section acquiring a reference pattern that corresponds to a structure of the object in a given state as the known characteristic information, and
the classification processing section generating a corrected pattern as the classification reference, and performing the classification process using the generated classification reference, the corrected pattern being acquired by performing a deformation process based on the surface shape information on the reference pattern.

19. The image processing device as defined in claim 18,
the classification processing section calculating a similarity between the structure of the object captured within the captured image and the corrected pattern used as the classification reference at each position within the captured image, and performing the classification process based on the calculated similarity.

20. The image processing device as defined in claim 18,
the known characteristic information acquisition section acquiring the reference pattern that corresponds to the structure of the object in a normal state as the known characteristic information.

21. The image processing device as defined in claim 20,
the known characteristic information acquisition section acquiring the reference pattern that corresponds to the structure of the object in an abnormal state as the known characteristic information.

22. The image processing device as defined in claim 17,
the known characteristic information acquisition section acquiring a reference pattern that corresponds to a structure of the object in a given state as the known characteristic information, and
the classification processing section performing a deformation process based on the surface shape information on the reference pattern to acquire a corrected pattern, calculating a similarity between the structure of the object captured within the captured image and the corrected pattern at each position within the captured image, acquiring a second reference pattern candidate based on the calculated similarity, generating a second reference pattern as a new reference pattern based on the acquired second reference pattern candidate and the surface shape information, performing the deformation process based on the surface shape information on the second reference pattern to generate a second corrected pattern as the classification reference, and performing the classification process using the generated classification reference.

23. The image processing device as defined in claim 17,
the object including a global three-dimensional structure, and a local concavity-convexity structure that is more local than the global three-dimensional structure, and
the surface shape calculation section calculating the surface shape information by extracting the global three-dimensional structure included in the object from the distance information without extracting the local concavity-convexity structure included in the object.

24. The image processing device as defined in claim 23,
the surface shape calculation section calculating a normal vector to a surface of the object represented by the global three-dimensional structure as the surface shape information.

25. The image processing device as defined in claim 24,
the known characteristic information acquisition section acquiring a reference pattern that corresponds to a structure of the object in a given state as the known characteristic information, and
the classification processing section generating a corrected pattern as the classification reference, and performing the classification process using the generated classification reference, the corrected pattern being acquired by performing a deformation process based on an angle of the normal vector with respect to a given reference direction on the reference pattern.

26. An electronic device comprising the image processing device as defined in any one of claims 1 to 25.

27. An endoscope apparatus comprising the image processing device as defined in any one of claims 1 to 25.

28. A program that causes a computer to perform steps of:

acquiring a captured image that includes an image of an object, the captured image being an image captured by an imaging section;
acquiring distance information based on a distance from the imaging section to the object when the imaging section captured the captured image;
acquiring known characteristic information, the known characteristic information being information that represents known characteristics relating to a structure of the object; and
performing a concavity-convexity determination process that specifies an concavity-convexity part of the object that agrees with characteristics specified by the known characteristic information, from the object captured within the captured image, based on the distance information and the known characteristic information.

29. An image processing method comprising:

acquiring a captured image that includes an image of an object, the captured image being an image captured by an imaging section;

acquiring distance information based on a distance from the imaging section to the object when the imaging section captured the captured image;

acquiring known characteristic information, the known characteristic information being information that represents known characteristics relating to a structure of the object; and

performing a concavity-convexity determination process that specifies an concavity-convexity part of the object that agrees with characteristics specified by the known characteristic information, from the object captured within the captured image, based on the distance information and the known characteristic information.

# FIG. 1

# FIG. 2

FIG. 3

FIG. 4A

SURFACE OF TISSUE

DISTANCE

200

FIG. 4B

CLOSING PROCESS

FIG. 4C

DETECTION OF CONCAVITIES

FIG. 4D

OPENING PROCESS

FIG. 4E

DETECTION OF CONVEXITIES

FIG. 4F

CHANGE DIAMETER (RADIUS) OF
SPHERE CORRESPONDING TO DISTANCE

O ——→ o ——→ o

PART OF DISTANCE MAP
OBJECT IS CAPTURED MORE DARKLY AS DISTANCE INCREASES,
AND CAPTURED MORE BRIGHTLY AS DISTANCE DECREASES

# FIG. 5

EP 2 937 033 A1

```
                              ┌──────────────┐
                              │   CONTROL    │ ⌒302
                              │   SECTION    │
                              └──────┬───────┘
                                     │
                                     ▼
                              ┌──────────────┐
                              │    KNOWN     │
                              │ CHARACTERISTIC│ ⌒350
                              │ INFORMATION  │
                              │ACQUISITION SECTION│
                              └──────┬───────┘
```

STEREO MATCHING SECTION — 341

PARALLAX-DISTANCE CONVERSION SECTION — 342

DISTANCE INFORMATION ACQUISITION SECTION — 340

LOCAL AVERAGE DISTANCE CALCULATION SECTION — 361

MORPHOLOGICAL CHARACTERISTIC SETTING SECTION — 362

CLOSING PROCESSING SECTION — 363-1

CONCAVITY EXTRACTION SECTION — 364

OPENING PROCESSING SECTION — 363-2

CONVEXITY EXTRACTION SECTION — 365

CONCAVITY-CONVEXITY INFORMATION EXTRACTION SECTION — 360

# FIG. 6

FIG. 7

302 CONTROL SECTION

350 KNOWN CHARACTERISTIC INFORMATION ACQUISITION SECTION

200 IMAGING SECTION

303 DISTANCE MAP STORAGE SECTION

360 CONCAVITY-CONVEXITY INFORMATION EXTRACTION SECTION

370 DETERMINATION PROCESSING SECTION

CONCAVITY-CONVEXITY DETERMINATION SECTION

310

390 IMAGE ACQUISITION SECTION

320 IMAGE CONSTRUCTION SECTION

330 ENHANCEMENT PROCESSING SECTION

400 DISPLAY SECTION

IMAGE PROCESSING SECTION

301

EP 2 937 033 A1

39

## FIG. 8A

SURFACE OF TISSUE

DISTANCE

## FIG. 8B

LOW-PASS FILTERING RESULTS

SUBTRACTION

## FIG. 8C

## FIG. 8D

CHANGE CHARACTERISTICS OF
LOW-PASS FILTER CORRESPONDING TO DISTANCE

RESPONSE          RESPONSE          RESPONSE

f                    f                    f

PART OF DISTANCE MAP
OBJECT IS CAPTURED MORE DARKLY AS DISTANCE INCREASES,
AND CAPTURED MORE BRIGHTLY AS DISTANCE DECREASES

FIG. 9

CONTROL SECTION /302

KNOWN CHARACTERISTIC INFORMATION ACQUISITION SECTION /350

303

DISTANCE MAP STORAGE SECTION

361

LOCAL AVERAGE DISTANCE CALCULATION SECTION

366

LOW-PASS CHARACTERISTIC SETTING SECTION

367

LOW-PASS PROCESSING SECTION

364

CONCAVITY EXTRACTION SECTION

365

CONVEXITY EXTRACTION SECTION

CONCAVITY-CONVEXITY INFORMATION EXTRACTION SECTION

360

EP 2 937 033 A1

# FIG. 10

FIG. 11

EP 2 937 033 A1

EP 2 937 033 A1

# FIG. 12

CONTROL SECTION — 302

KNOWN CHARACTERISTIC INFORMATION ACQUISITION SECTION — 350

601 — DISTANCE INFORMATION ACQUISITION SECTION

361 — LOCAL AVERAGE DISTANCE CALCULATION SECTION

368 — HIGH-PASS CHARACTERISTIC SETTING SECTION

369 — HIGH-PASS PROCESSING SECTION

364 — CONCAVITY EXTRACTION SECTION

365 — CONVEXITY EXTRACTION SECTION

CONCAVITY-CONVEXITY INFORMATION EXTRACTION SECTION

360

# FIG. 13

303

DISTANCE MAP
STORAGE
SECTION

390

IMAGE
ACQUISITION
SECTION

602

STEREO
MATCHING
SECTION

342

PARALLAX-
DISTANCE
CONVERSION
SECTION

DISTANCE INFORMATION
ACQUISITION SECTION

601

FIG. 14

EP 2 937 033 A1

# FIG. 15

# FIG. 16

EP 2 937 033 A1

FIG. 17A

FIG. 17B

# FIG. 18A

CLOSING PROCESS

*DESIGNATE RADIUS THAT
IS LARGER THAN SIZE
OF PIT PATTERN

DISTANCE

SPHERE HAVING GIVEN
RADIUS USED FOR
CLOSING PROCESS

~200

SURFACE OF TISSUE

# FIG. 18B

CALCULATE NORMAL VECTOR WITH RESPECT TO RESULTS OF CLOSING PROCESS

NORMAL VECTOR

50

FIG. 19A

FIG. 19B

BASIC PIT

CORRECTED PIT

# FIG. 20

EP 2 937 033 A1

FIG. 21

EP 2 937 033 A1

# FIG. 22

AREA THAT IS
NOT NORMAL

AREA THAT IS
NOT NORMAL

NORMAL AREA

# FIG. 23

FIG. 24

330 ENHANCEMENT PROCESSING SECTION

385 CLASSIFICATION PROCESSING SECTION

3855 AREA SETTING SECTION

3856 SECOND CLASSIFICATION REFERENCE DATA GENERATION SECTION

3854 SIMILARITY CALCULATION SECTION

320 IMAGE CONSTRUCTION SECTION

350 CONTROL SECTION

3851 CLASSIFICATION REFERENCE DATA STORAGE SECTION

3852 PROJECTIVE TRANSFORMATION SECTION

3853 SEARCH AREA SIZE SETTING SECTION

380 SURFACE SHAPE CALCULATION SECTION

340 DISTANCE INFORMATION ACQUISITION SECTION

# FIG. 25

| CLASS | PIT |
|---|---|
| TYPE I | TYPE A |
| TYPE II | TYPE B |
| | TYPE C |
| TYPE III | TYPE D |
| | TYPE E |
| | TYPE F |
| ... | ... |

FIG. 26A

TYPE I – TYPE A

FIG. 26B

TYPE II – TYPE C

FIG. 26C

TYPE III – TYPE F

FIG. 26D

TYPE III

TYPE II

TYPE I

EP 2 937 033 A1

## FIG. 27A

PROCESSING
TARGET POSITION

CAPTURED IMAGE

## FIG. 27B

CORRECTED
PATTERN

## FIG. 27C

PROCESSING
TARGET POSITION

SEARCH AREA

## FIG. 27D

SEARCH AREA

## FIG. 27E

SIMILARITY
VALUE PEAK

AREA THAT
AGREES WITH
CLASSIFICATION
REFERENCE

SEARCH AREA

## FIG. 27F

SIMILARITY
VALUE PEAK

AREA THAT
AGREES WITH
CLASSIFICATION
REFERENCE

SEARCH AREA

FIG. 28A

I

FIG. 28B

II

FIG. 28C

IIIs

FIG. 28D

IIIL

FIG. 28E

IV

FIG. 28F

V

## INTERNATIONAL SEARCH REPORT

| | International application No. |
|---|---|
| | PCT/JP2013/075870 |

**A. CLASSIFICATION OF SUBJECT MATTER**
*A61B1/00*(2006.01)i, *G02B23/24*(2006.01)i, *G06T1/00*(2006.01)i, *H04N7/18* (2006.01)i

According to International Patent Classification (IPC) or to both national classification and IPC

**B. FIELDS SEARCHED**

Minimum documentation searched (classification system followed by classification symbols)
A61B1/00, G02B23/24, G06T1/00, H04N7/18

Documentation searched other than minimum documentation to the extent that such documents are included in the fields searched
Jitsuyo Shinan Koho 1922–1996 Jitsuyo Shinan Toroku Koho 1996–2013
Kokai Jitsuyo Shinan Koho 1971–2013 Toroku Jitsuyo Shinan Koho 1994–2013

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2011-200572 A (Fujifilm Corp.), 13 October 2011 (13.10.2011), paragraphs [0003], [0044] (Family: none) | 1-28 |
| A | JP 2010-68865 A (Fujifilm Corp.), 02 April 2010 (02.04.2010), paragraphs [0096] to [0115]; fig. 13, 14 & US 2010/0069747 A1 & EP 2163191 A1 | 1-28 |
| A | JP 2003-88498 A (Pentax Corp.), 25 March 2003 (25.03.2003), paragraph [0050] (Family: none) | 1-28 |

☒ Further documents are listed in the continuation of Box C.  ☐ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search <br> 19 December, 2013 (19.12.13) | Date of mailing of the international search report <br> 07 January, 2014 (07.01.14) |
|---|---|
| Name and mailing address of the ISA/ <br> Japanese Patent Office | Authorized officer |
| Facsimile No. | Telephone No. |

Form PCT/ISA/210 (second sheet) (July 2009)

## INTERNATIONAL SEARCH REPORT

International application No.

PCT/JP2013/075870

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2009-273655 A (Fujifilm Corp.),<br>26 November 2009 (26.11.2009),<br>paragraph [0087]; fig. 7<br>(Family: none) | 1-28 |

Form PCT/ISA/210 (continuation of second sheet) (July 2009)

**INTERNATIONAL SEARCH REPORT**

| International application No. |
|---|
| PCT/JP2013/075870 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 29
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

| International application No. |
| --- |
| PCT/JP2013/075870 |

Continuation of Box No.II-1 of continuation of first sheet(2)

The invention relates to a method which involves insertion of an endoscope into the human body and is hence conducted during a treatment of the human body by surgery. Consequently, the invention relates to a subject matter for which the International Searching Authority is not required, under the provisions of PCT Article 17(2)(a)(i) and PCT Rule 39.1(iv), to make a search.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003088498 A

- JP 2010068865 A